# EUROPEAN PATENT SPECIFICATION

(11) **EP 1 311 544 B1**
(45) Date of publication and mention of the grant of the patent: **04.07.2007**
(21) Application number: 01922808.9
(22) Date of filing: 28.03.2001
(51) Int. Cl.: C07K 14/705, C12N 15/12

(54) **HUMAN 7-TRANSMEMBRANE PROTEINS AND POLYNUCLEOTIDES ENCODING THE SAME**
HUMANE 7-TRANSMEMBRANPROTEINE UND DAFÜR CODIERENDE POLYNUKLEOTIDE
NOUVELLES PROTEINES HUMAINES 7TM ET POLYNUCLEOTIDES CODANT POUR CES PROTEINES

(30) Priority: 28.03.2000 US 192978 P
(43) Date of publication of application: 21.05.2003
(73) Proprietor: Lexicon Genetics Incorporated, The Woodlands, TX 77381-1160 (US)
(72) Inventor: WALKE, D., Wade, Spring, TX 77389 (US); SCOVILLE, John, Pearland, TX 77584 (US); DONOHO, Gregory, Portage, MI 49024 (US); TURNER, C., Alexander, Jr., The Woodlands, TX 77382 (US); FRIEDRICH, Glenn c/o BCM Technologies, Houston, TX 77046 (US); ABUIN, Alejandro, The Woodlands, TX 77382 (US); ZAMBROWICZ, Brian, The Woodlands, TX 77382 (US); SANDS, Arthur, T., The Woodlands, TX 77381 (US)
(74) Representative: Silveston, Judith
(86) International application number: PCT/US2001/009996
(87) International publication number: WO 2001/072842

(56) References cited:
- WO-A-00/06592
- VALVE, R. ET AL.: "Two polymorphisms in the peroxisome proliferator-activated receptor-gamma gene are associated with severe overweight among obese women. polymorphisms in the PPARgamma gene are associated with severe overweight and increased fat mass among obese" THE JOURNAL OF CLINICAL ENDOCRINOLOGY AND METABOLISM, vol. 84, no. 10, October 1999 (1999-10), pages 3708-3712, XP002348946

## Description

The present application claims the benefit of U.S. Provisional Application Number 60/192,978 which was filed on March 28, 2000.

### 1. INTRODUCTION

The present invention relates to the discovery, identification and characterization of novel human polynucleotides that encode membrane associated proteins and receptors. The invention encompasses the described polynucleotides, host cell expression systems, the encoded proteins, fusion proteins, polypeptides and peptides, antibodies to the encoded proteins and peptides, and genetically engineered animals that lack the disclosed polynucleotides, or over express the disclosed polynucleotides, or antagonists and agonists of the proteins, and other compounds that modulate the expression or activity of the proteins encoded by the disclosed polynucleotides that can be used for diagnosis, drug screening, clinical trial monitoring, and/or the treatment of physiological or behavioral disorders.

### 2. BACKGROUND OF THE INVENTION

Membrane receptor proteins can serve as integral components of cellular mechanisms for sensing their environment, and maintaining cellular homeostasis and function. Accordingly, membrane receptor proteins are often involved in signal transduction pathways that control cell physiology, chemical communication, and gene expression. A particularly relevant class of membrane receptors are those typically characterized by the presence of 7 conserved transmembrane domains that are interconnected by nonconserved hydrophilic loops. Such, "7TM receptors" include a superfamily of receptors known as G-protein couples receptors (GPCR's). GPCRs are typically involved in signal transduction pathways involving G-proteins or PPG proteins. As such, the GPCR family includes many receptors that are known to serve as drug targets for therapeutic agents.

Valve et al., (1999) "Two polymorphisms in the peroxisome proliferator-activated receptor-gamma gene are associated with severe overweight among obese women" J. Clin. Endocrinol. 84(1)):3708-3712 discloses two polymorphisms in the PPARγ gene which are associated with increased weight in Finns.

WO 00/06592 discloses the sequences of nucleic acids encoding a G-Protein couples receptor involved in sensory transduction.

### 3. SUMMARY OF THE INVENTION

The present invention relates to the discovery, identification, and characterization of nucleotides that encode novel GPCRs, and the corresponding novel GPCR (NGPCR) amino acid sequences. The NGPCRs described for the first time herein are transmembrane proteins that span the cellular membrane and are involved in signal transduction after ligand binding. The described NGPCRs have structural motifs found in the 7TM receptor family. Expression of the described NGPCRs can be detected in human testis and kidney cells. The novel human GPCR sequences described herein encode proteins of 841, 763, 366, and 234 amino acids in length (see respectively SEQ ID NOS: 2, 4, 6, and 8). The described NGPCRs have a characteristic leader sequence, and contain multiple transmembrane regions (of about 20-30 amino acids) characteristic of 7TM proteins, as well as several predicted cytoplasmic domains.

Additionally contemplated are "knockout" ES cells that have been engineered using conventional methods (see, for example, PCT Applic. No. PCT/US98/03243, filed February 20, 1998). Accordingly, an additional aspect of the present invention includes knockout cells and animals having genetically engineered mutations in the polynucleotides encoding the presently described NGPCRs.

The invention encompasses the nucleotides presented in the Sequence Listing, host cells expressing such nucleotides, and the expression products of such nucleotides, and: (a) nucleotides that encode mammalian homologs of the described NGPCRs, including the specifically described human NGPCRs, and the human NGPCR gene products; (b) nucleotides that encode one or more portions of the NGPCRs that correspond to functional domains, and the polypeptide products specified by such nucleotide sequences, including but not limited to the novel regions of the described extracellular domain(s) (ECD), one or more transmembrane domain(s) (TM) first disclosed herein, and the cytoplasmic domain(s) (CD); (c) isolated nucleotides that encode mutants, engineered or naturally occurring, of the described NGPCRs in which all or a part of at least one of the domains is deleted or altered, and the polypeptide products specified by such nucleotide sequences, including but not limited to soluble receptors in which all or a portion of the TM is deleted, and nonfunctional receptors in which all or a portion of the CD is deleted; (d) nucleotides that encode fusion proteins containing the coding region from an NGPCR, or one of its domains (*e.g.*, an extracellular domain) fused to another peptide or polypeptide.

The invention also encompasses agonists and antagonists of the described NGPCRs, including small molecules, large molecules, mutant NGPCRs, or portions thereof, that compete with native NGPCR, peptides, and antibodies, as well as nucleotide sequences that can be used to inhibit the expression of the described NGPCRs (*e.g*., antisense and ribozyme molecules, and gene or regulatory sequence replacement constructs) or to enhance the expression of the described NGPCR polynucleotides (*e.g.*, expression constructs that place the described polynucleotide under the control of a strong promoter system), and transgenic animals that express a NGPCR transgene, or "knock-outs" (which can be conditional) that do not express a functional NGPCR. Knock-out mice can be produced in several ways, one or which involved the use of mouse embryonic stem cells ("ES cells") lines that contain gene trap mutations in a murine homolog of at least one of the described NGPCRs. When the unique NGPCR sequences described in SEQ ID NOS: 1-9 are "knocked-out" they provide a method of identifying phenotypic expression of the particular gene as well as a method of assigning function to previously unknown genes. Additionally, the unique NGPCR sequences described in SEQ ID NOS; 1-9 are useful for the identification of coding sequence and the mapping a unique gene to a particular chromosome.

Further, the present invention also relates to methods for the use of the described NGPCR gene and/or NGPCR gene products for the identification of compounds that modulate, i.e., act as agonists or antagonists, of NGPCR gene expression and or NGPCR gene product activity. Such compounds can be used as therapeutic agents for the treatment of various symptomatic representations of biological disorders or imbalances.

The invention also provides use of a nucleic acid molecule and polypeptides of the invention in a method of diagnosing a weight disorder, a method of screening drugs for use in the treatment of a weight disorder or a method of monitoring a clinical trial of a treatment for a weight disorder, subject to the proviso that methods for treatment of the human or animal body by surgery or therapy and diagnostic methods practised on the human or animal body are not claimed.

The invention also provides use according to the invention or an eptitope-binding fragment or derivative of said antibody, for example, a polyclonal or a monoclonal antibody, a single chain antibody, a Gab fragment, a F(ab')₂, a fragment produced by a Fab expression library or an anti-idiotypic (anti-Id) antibody, in a method of diagnosing a weight disorder, a method of screening drugs for use in the treatment of a weight disorder or a method of monitoring a clinical trial of a treatment for a weight disorder, subject to the proviso that methods for treatment of the human or animal body by surgery or therapy and diagnostic methods practised on the human or animal body are not claimed.

The invention also provides use of an antibody according to the invention or an epitope-binding fragment or derivative of said antibody, for example, a polyclonal or a monoclonal antibody, a single chain antibody, a Fab fragment, a F(ab')₂, a fragment produced by a Fab expression library, or an anti-idiotypic (anti-Id_ antibody, in the manufacture of a medicament for the treatment of a weight disorder.

The invention also provides an antisense nucleic acid molecule that inhibits the expression of a polypeptide according to the invention for use as a medicament for the treatment of a weight disorder; and use of an antisense nucleic acid molecule that inhibits expression of a polypeptide according to the invention in the manufacture of a medicament for the treatment of a weight disorder.

### 4. DESCRIPTION OF THE SEQUENCE LISTING AND FIGURES

The Sequence Listing provides the sequence of 4 NGPCR ORFs, the amino acid sequences encoded thereby, as well as an ORF with surrounding 5' and 3' regions (SEQ ID NO:9).

### 5. DETAILED DESCRIPTION OF THE INVENTION

The human NGPCRs, described for the first time herein, are novel receptor proteins that are expressed in human cells. The described NGPCR sequences were obtained using sequences from gene trapped human cells and cDNA clones isolated from human kidney and testis cDNA libraries (Edge Biosystems, Gaithersburg, MD, and Clontech, Palo Alto, CA). The described NGPCRs are transmembrane proteins that fall within the 7TM family of receptors. As with other GPCRs, signal transduction is triggered when a ligand binds to the receptor. Interfering with the binding of the natural ligand, or neutralizing or removing the ligand, or interference with its binding to a NGPCR will effect NGPCR mediated signal transduction. Because of their biological significance, 7TM, and particularly GPCR, proteins have been subjected to intense scientific/commercial scrutiny (see, for example, U.S. Applic. Ser. Nos. 08/820,521, filed March 19, 1997, and 08/833,226, filed April 17, 1997 for applications, uses, and assays involving the described NGPCRs). The presently described NGPCRs share significant homology with mammalian taste and pheromone receptors, calcium sensing receptors, and peptide hormone receptors.

The described NGPCRs also share similarity with metabotropic amino acid, or glutamate, receptors. Metabotropic glutamate receptors have been implicated in neurodegeneration, seizures, schizophrenia, and other neural or behavioral disorders. As such, this subclass of receptors have been subject to considerable study as evidenced by U.S. Patents Nos. 5,869,609, 5,912,122, 5,981,195, and 6,001,581, and U.S. Application Ser. No. 60/085,973 which describe related compositions and assays and uses therefor.

The invention encompasses the use of the described NGPCR nucleotides, NGPCR proteins and peptides, as well as antibodies, preferably humanized monoclonal antibodies, or binding fragments, domains, or fusion proteins thereof, to the NGPCRs (which can, for example, act as NGPCR agonists or antagonists), antagonists that inhibit receptor activity or expression, or agonists that activate receptor activity or increase its expression in the diagnosis and treatment of disease.

In particular, the invention described in the subsections below encompasses NGPCR polypeptides or peptides corresponding to functional domains of NGPCR (e.g., ECD, TM or CD), mutated, truncated or deleted NGPCRs (e.g., NGPCRs missing one or more functional domains or portions thereof, such as, ΔECD, ΔTM and/or ΔCD), NGPCR fusion proteins (e.g., a NGPCR or a functional domain of a NGPCR, such as the ECD, fused to an unrelated protein or peptide such as an immunoglobulin constant region, i.e., IgFc), nucleotide sequences encoding such products, and host cell expression systems that can produce such NGPCR products.

The invention also encompasses antibodies and anti-idiotypic antibodies (including Fab fragments), antagonists and agonists of the NGPCR, as well as compounds or nucleotide constructs that inhibit expression of a NGPCR gene (transcription factor inhibitors, antisense and ribozyme molecules, or gene or regulatory sequence replacement constructs), or promote expression of NGPCR (e.g., expression constructs in which NGPCR coding sequences are operatively associated with expression control elements such as promoters, promoter/enhancers, etc.). The invention also relates to host cells and animals genetically engineered to express the human NGPCRs (or mutants thereof) or to inhibit or "knock-out" expression of the animal's endogenous NGPCR genes.

The NGPCR proteins or peptides, NGPCR fusion proteins, NGPCR nucleotide sequences, antibodies, antagonists and agonists can be useful for the detection of mutant NGPCRs or inappropriately expressed NGPCRs for the diagnosis of disease. The NGPCR proteins or peptides, NGPCR fusion proteins, NGPCR nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NGPCR in the body. The use of engineered host cells and/or animals may offer an advantage in that such systems allow not only for the identification of compounds that bind to an ECD of a NGPCR, but can also identify compounds that affect the signal transduced by an activated NGPCR.

Finally, the NGPCR protein products (especially soluble derivatives such as peptides corresponding to the NGPCR ECD, or truncated polypeptides lacking on or more TM domains) and fusion protein products (especially NGPCR-Ig fusion proteins, *i.e*., fusions of a NGPCR, or a domain of a NGPCR, *e.g*., ECD, ΔTM to an IgFc), antibodies and anti-idiotypic antibodies (including Fab fragments), antagonists or agonists (including compounds that modulate signal transduction which may act on downstream targets in a NGPCR-mediated signal transduction pathway) can be used for therapy of such diseases. For example, the administration of an effective amount of soluble NGPCR ECD, ΔTM, or an ECD-IgFc fusion protein or an anti-idiotypic antibody (or its Fab) that mimics the NGPCR ECD would "mop up" or "neutralize" the endogenous NGPCR ligand, and prevent or reduce binding and receptor activation. Nucleotide constructs encoding such NGPCR products can be used to genetically engineer host cells to express such products *in vivo*; these genetically engineered cells function as "bioreactors" in the body delivering a continuous supply of a NGPCR, a NGPCR peptide, soluble ECD or ΔTM or a NGPCR fusion protein that will "mop up" or neutralize a NGPCR ligand. Nucleotide constructs encoding functional NGPCRs, mutant NGPCRs, as well as antisense and ribozyme molecules can be used in "gene therapy" approaches for the modulation of NGPCR expression. Thus, the invention also encompasses pharmaceutical formulations.

Various aspects of the invention are described in greater detail in the subsections below.

### 5.1 THE NGPCR POLYNUCLEOTIDES

The cDNA sequences and deduced amino acid sequences of the described human NGPCRs are presented in the Sequence Listing. Several polymorphisms were identified during the sequencing of the described NGPCRs including: a C or T transition at the position represented by, for example, nucleotide 320 of SEQ ID NO:1 which results in a corresponding 5 (preferred) or F being present at amino acid position 107 of, for example, SEQ ID NO:2; an A or G transition at the position represented by, for example, nucleotide 1,114 of SEQ ID NO.1 which results in a corresponding A (preferred) or T being present at amino acid position 372 of, for example, SEQ ID NO:2; and a silent C or T transition at the position represented by, for example, nucleotide 2526 of SEQ ID NO:1. Where applicable, regions of SEQ ID NOS 3-8 that correspond to the above mentioned regions can also display the described polymorphisms.

The NGPCRs of the present invention include: (a) the human DNA sequences presented in the Sequence Listing and additionally contemplate any nucleotide sequence encoding a contiguous and functional NGPCR open reading frame (ORF) that hybridizes to a complement of the DNA sequences presented in the Sequence Listing under highly stringent conditions, *e.g.,* hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et al., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of DNA sequences that encode and express an amino acid sequence presented in the Sequence Listing under moderately stringent conditions, *e.g*., washing in 0.2xSSC/0.1% SDS at 42°C (Ausubel et al., 1989, *supra),* yet which still encode a functionally equivalent NGPCR gene product. Functional equivalents of NGPCR include naturally occurring NGPCRs present in other species, and mutant NGPCRs whether naturally occurring or engineered. The invention also includes degenerate variants of the disclosed sequences.

Additionally contemplated are polynucleotides encoding NGPCR ORFs, or their functional equivalents, encoded by polynucleotide sequences that are about 99, 95, 90, or about 85 percent similar or identical to corresponding regions of the polynucleotide sequences described in the Sequence Listing (as measured by BLAST sequence comparison analysis using, for example, the GCG sequence analysis package using default parameters).

The invention also includes nucleic acid molecules, preferably DNA molecules, that hybridize to, and are therefore the complements of, the described NGPCR nucleotide sequences. Such hybridization conditions may be highly stringent or less highly stringent, as described above. In instances wherein the nucleic acid molecules are deoxyoligonucleotides ("DNA oligos"), such molecules (and particularly about 16 to about 100 base long, about 20 to about 80, or about 34 to about 45 base long, or any variation or combination of sizes represented therein incorporating a contiguous region of sequence first disclosed in the present Sequence Listing, can be used in conjunction with the polymerase chain reaction (PCR) to screen libraries, isolate clones, and prepare cloning and sequencing templates, etc.

Alternatively, such NGPCR oligonucleotides can be used as hybridization probes for screening libraries, and assessing gene expression patterns (particularly using a micro array or high-throughput "chip" format). Additionally, a series of the described NGPCR oligonucleotide sequences, or the complements thereof, can be used to represent all or a portion of the described NGPCR sequences. An oligonucleotide or polynucleotide sequence first disclosed in at least a portion of one or more of the sequences of SEQ ID NOS: 1-9 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (*i.e*., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-9, or an amino acid sequence encoded thereby. Methods for attaching biopolymers to, or synthesizing biopolymers on, solid support matrices, and conducting binding studies thereon are disclosed in, inter alia, U.S. Patent Nos. 5,700,637, 5,556,752, 5,744,305, 4,631,211, 5,445,934, 5,252,743, 4,713,326, 5,424,186, and 4,689,405 the disclosures of which are herein incorporated by reference in their entirety.

Addressable arrays comprising sequences first disclosed in SEQ ID NOS:1-9 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-9.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-9 provides detailed information about transcriptional changes involved in a specific pathway, potentially leading to the identification of novel components or gene functions that manifest themselves as novel phenotypes.

Probes consisting of sequences first disclosed in SEQ ID NOS:1-9 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-9 can be utilized in microarrays or other assay formats, to screen collections of genetic material from patients who have a particular medical condition. These investigations can also be carried out using the sequences first disclosed in SEQ ID NOS:1-9 *in silico* and by comparing previously collected genetic databases and the disclosed sequences using computer software known to those in the art.

Thus the sequences first disclosed in SEQ ID NOS:1-9 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-9. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (*e.g*., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relatve to one or more additional sequence(s) or one or more restriction sites present in the disclosed sequence. For oligonucleotides probes, highly stringent conditions may refer, *e.g*., to washing in 6xSSC/0.05% sodium pyrophosphate at 37°C (for 14-base oligos), 48°C (for 17-base oligos), 55°C (for 20-base oligos), and 60°C (for 23-base oligos).

The described oligonucleotides may encode or act as NGPCR antisense molecules, useful, for example, in NGPCR gene regulation (for and/or as antisense primers in amplification reactions of NGPCR gene nucleic acid sequences). With respect to NGPCR gene regulation, such techniques can be used to regulate biological functions. Further, such sequences may be used as part of ribozyme and/or triple helix sequences, also useful for NGPCR gene regulation.

Additionally, the antisense oligonucleotides may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-0-methylribonucleotide (Inoue et al., 1987, Nucl. Acids Res. 15:6131-6148), or a chimeric RNA-DNA analogue (Inoue et al., 1987, FEBS Lett. 215:327-330).

Oligonucleotides of the invention may be synthesized by standard methods known in the art, *e.g*. by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook et al., 1989, Molecular Cloning, A Laboratory Manual (and periodic updates thereof), Cold Springs Harbor Press, N.Y.; and Ausubel et al., 1989, Current Protocols in Molecular Biology, Green Publishing Associates and Wiley Interscience, N.Y.

Alternatively, suitably labeled NGPCR nucleotide probes may be used to screen a human genomic library using appropriately stringent conditions or by PCR. The identification and characterization of human genomic clones is helpful for identifying polymorphisms, determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g*., splice acceptor and/or donor sites), etc., that can be used in diagnostics and pharmacogenomics.

Further, a NGPCR gene homolog may be isolated from nucleic acid of the organism of interest by performing PCR using two degenerate oligonucleotide primer pools designed on the basis of amino acid sequences within the NGPCR gene product disclosed herein. The template for the reaction may be total RNA, mRNA, and/or cDNA obtained by reverse transcription of mRNA prepared from, for example, human or non-human cell lines or tissue known or suspected to express a NGPCR gene allele.

The PCR product may be subcloned and sequenced to ensure that the amplified sequences represent the sequence of the desired NGPCR gene. The PCR fragment may then be used to isolate a full length cDNA clone by a variety of methods. For example, the amplified fragment may be labeled and used to screen a cDNA library, such as a bacteriophage cDNA library. Alternatively, the labeled fragment may be used to isolate genomic clones via the screening of a genomic library.

PCR technology may also be utilized to isolate full length cDNA sequences. For example, RNA may be isolated, following standard procedures, from an appropriate cellular or tissue source (*i.e*., one known, or suspected, to express a NGPCR gene). A reverse transcription (RT) reaction may be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment may easily be isolated. For a review of cloning strategies which may be used, see *e.g.,* Sambrook *et al.,* 1989, *supra.*

A cDNA of a mutant NGPCR gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NGPCR allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NGPCR allele to that of the normal NGPCR allele, the mutation(s) responsible for the loss or alteration of function of the mutant NGPCR gene product can be ascertained.

Alternatively, a genomic library can be constructed using DNA obtained from an individual suspected of or known to carry the mutant NGPCR allele, or a cDNA library can be constructed using RNA from a tissue known, or suspected, to express the mutant NGPCR allele. A normal NGPCR gene, or any suitable fragment thereof, can then be labeled and used as a probe to identify the corresponding mutant NGPCR allele in such libraries. Clones containing the mutant NGPCR gene sequences can then be purified and subjected to sequence analysis according to methods well known to those of skill in the art.

Additionally, an expression library can be constructed utilizing cDNA synthesized from, for example, RNA isolated from a tissue known, or suspected, to express a mutant NGPCR allele in an individual suspected of or known to carry such a mutant allele. In this manner, gene products made by the putatively mutant tissue may be expressed and screened using standard antibody screening techniques in conjunction with antibodies raised against the normal NGPCR gene product, as described, below, in Section 5.3. (For screening techniques, see, for example, Harlow, E. and Lane, eds., 1988, "Antibodies: A Laboratory Manual", Cold Spring Harbor Press, Cold Spring Harbor, NY.)

Additionally, screening can be accomplished by screening with labeled NGPCR fusion proteins; such as, for example, alkaline phosphatase-NGPCR or NGPCR-alkaline phosphatase fusion proteins. In cases where a NGPCR mutation results in an expressed gene product with altered function (*e.g.*, as a result of a missense or a frameshift mutation), a polyclonal set of antibodies to NGPCR are likely to cross-react with the mutant NGPCR gene product. Library clones detected via their reaction with such labeled antibodies can be purified and subjected to sequence analysis according to methods well known to those of skill in the art.

The invention also encompasses nucleotide sequences that encode mutant NGPCRs, peptide fragments of the NGPCRs, truncated NGPCRs, and NGPCR fusion proteins. These include, but are not limited to, nucleotide sequences encoding mutant NGPCRs described below; polypeptides or peptides corresponding to one or more ECD, TM and/or CD domains of the NGPCR or portions of these domains; truncated NGPCRs in which one or two of the domains is deleted, *e.g.*, a soluble NGPCR lacking the TM or both the TM and CD regions, or a truncated, nonfunctional NGPCR lacking all or a portion of the CD region. Nucleotides encoding fusion proteins may include, but are not limited to, full length NGPCR sequences, truncated NGPCRs, or nucleotides encoding peptide fragments of NGPCR fused to an unrelated protein or peptide, such as for example, a transmembrane sequence, which anchors the NGPCR ECD to the cell membrane; an IgFc domain which increases the stability and half life of the resulting fusion protein (*e.g*., NGPCR-Ig) in the bloodstream; or an enzyme, fluorescent protein, luminescent protein which can be used as a marker.

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patents Nos. 5,830,721 and 5,837,458.

The invention also encompasses (a) DNA vectors that contain any of the foregoing NGPCR coding sequences and/or their complements (*i.e*., antisense); (b) DNA expression vectors that contain any of the foregoing NGPCR coding sequences operatively associated with a regulatory element that directs the expression of the coding sequences; and (c) genetically engineered host cells that contain any of the foregoing NGPCR coding sequences operatively associated with a regulatory element that directs the expression of the coding sequences in the host cell. As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

### 5.2 NGPCR PROTEINS AND POLYPEPTIDES

NGPCR proteins, polypeptides and peptide fragments, mutated, truncated or deleted forms of the NGPCR and/or NGPCR fusion proteins can be prepared for a variety of uses. These uses include, but are not limited to the generation of antibodies, as reagents in diagnostic assays, the identification of other cellular gene products related to a NGPCR, as reagents in assays for screening for compounds that can be used as pharmaceutical reagents useful in the therapeutic treatment of mental, biological, or medical disorders (*i.e*., heartbeat rate, etc.) and disease.

The Sequence Listing discloses the amino acid sequences encoded by the described NGPCR polynucleotides. The NGPCRs have initiator methionines in DNA sequence contexts consistent with translation initiation sites, followed by hydrophobic signal sequences typical of membrane associated proteins. The sequence data presented herein indicate that alternatively spliced forms of the NGPCRs exist (which may or may not be tissue specific).

The NGPCR amino acid sequences of the invention include the nucleotide and amino acid sequences presented in the Sequence Listing as well as analogues and derivatives thereof. Further, corresponding NGPCR homologues from other species are encompassed by the invention. In fact, any NGPCR protein encoded by the NGPCR nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell et al. eds., Scientific American Books, New York, NY ) are generically representative of all the various permutations and combinations of nucleic acid sequences that can encode such amino acid sequences.

The invention also encompasses proteins that are functionally equivalent to the NGPCR encoded by the described nucleotide sequences as judged by any of a number of criteria, including but not limited to the ability to bind a ligand for a NGPCR, the ability to effect an identical or complementary signal transduction pathway, a change in cellular metabolism (*e.g*., ion flux, tyrosine phosphorylation, etc.) or change in phenotype when the NGPCR equivalent is present in an appropriate cell type (such as the amelioration, prevention or delay of a biochemical, biophysical, or overt phenotype. Such functionally equivalent NGPCR proteins include but are not limited to additions or substitutions of amino acid residues within the amino acid sequence encoded by the NGPCR nucleotide sequences described above but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

While random mutations can be made to NGPCR DNA (using random mutagenesis techniques well known to those skilled in the art) and the resulting mutant NGPCRs tested for activity, site-directed mutations of the NGPCR coding sequence can be engineered (using site-directed mutagenesis techniques well known to those skilled in the art) to generate mutant NGPCRs with increased function, *e.g.*, higher binding affinity for the target ligand, and/or greater signaling capacity; or decreased function, and/or decreased signal transduction capacity. One starting point for such analysis is by aligning the disclosed human sequences with corresponding gene/protein sequences from, for example, other mammals in order to identify amino acid sequence motifs that are conserved between different species. Non-conservative changes can be engineered at variable positions to alter function, signal transduction capability, or both. Alternatively, where alteration of function is desired, deletion or non-conservative alterations of the conserved regions (*i.e*., identical amino acids) can be engineered. For example, deletion or non-conservative alterations (substitutions or insertions) of the various conserved transmembrane domains.

An additional application of the described NGPCR polynucleotide sequences is their use in the molecular mutagenesis/evolution of proteins that are at least partially encoded by the described novel sequences using, for example, polynucleotide shuffling or related methodologies. Such approaches are described in U.S. Patents Nos. 5,830,721 and 5,837,458.

Additionally contemplated uses for the described sequences include the engineering of constitutively "on" variants for use in cell assays and genetically engineered animals using the methods and applications described in U.S. Patent Applications Ser Nos. 60/110,906, 60/106,300, 60/094,879, and 60/121,851.

Other mutations in the NGPCR coding sequence can be made to generate NGPCRs that are better suited for expression, scale up, etc. in the host cells chosen. For example, cysteine residues can be deleted or substituted with another amino acid in order to eliminate disulfide bridges; N-linked glycosylation sites can be altered or eliminated to achieve, for example, expression of a homogeneous product that is more easily recovered and purified from yeast hosts which are known to hyperglycosylate N-linked sites. To this end, a variety of amino acid substitutions at one or both of the first or third amino acid positions of any one or more of the glycosylation recognition sequences which occur in the ECD (N-X-S or N-X-T), and/or an amino acid deletion at the second position of any one or more such recognition sequences in the ECD will prevent glycosylation of the NGPCR at the modified tripeptide sequence. (See, *e.g.,* Miyajima et al., 1986, EMBO J. 5(6):1193-1197).

Peptides corresponding to one or more domains of the NGPCR (*e.g*., ECD, TM, CD, etc.), truncated or deleted NGPCRs (*e.g.*, NGPCR in which a ECD, TM and/or CD is deleted) as well as fusion proteins in which a full length NGPCR, a NGPCR peptide, or truncated NGPCR is fused to an unrelated protein, are also within the scope of the invention and can be designed on the basis of the presently disclosed NGPCR nucleotide and NGPCR amino acid sequences. Such fusion proteins include but are not limited to IgFc fusions which stabilize the NGPCR protein or peptide and prolong half-life *in vivo;* or fusions to any amino acid sequence that allows the fusion protein to be anchored to the cell membrane, allowing an ECD to be exhibited on the cell surface; or fusions to an enzyme, fluorescent protein, or luminescent protein which provide a marker function.

While the NGPCR polypeptides and peptides can be chemically synthesized (*e.g*., see Creighton, 1983, Proteins: Structures and Molecular Principles, W.H. Freeman & Co., N.Y.), large polypeptides derived from a NGPCR and full length NGPCRs can be advantageously produced by recombinant DNA technology using techniques well known in the art for expressing nucleic acid containing NGPCR gene sequences and/or coding sequences. Such methods can be used to construct expression vectors containing a presently described NGPCR nucleotide sequences and appropriate transcriptional and translational control signals. These methods include, for example, *in vitro* recombinant DNA techniques, synthetic techniques, and in vivo genetic recombination. See, for example, the techniques described in Sambrook *et al.,* 1989, *supra,* and Ausubel *et al.*, 1989, supra. Alternatively, RNA corresponding to all or a portion of a transcript encoded by a NGPCR nucleotide sequence may be chemically synthesized using, for example, synthesizers. See, for example, the techniques described in "Oligonucleotide Synthesis", 1984, Gait, M.J. ed., IRL Press, Oxford.

A variety of host-expression vector systems may be utilized to express the NGPCR nucleotide sequences of the invention. Where the NGPCR peptide or polypeptide is a soluble derivative (*e.g*., NGPCR peptides corresponding to an ECD; truncated or deleted NGPCR in which a TM and/or CD are deleted) the peptide or polypeptide can be recovered from the culture, *i.e*., from the host cell in cases where the NGPCR peptide or polypeptide is not secreted, and from the culture media in cases where the NGPCR peptide or polypeptide is secreted by the cells. However, such expression systems also encompass engineered host cells that express a NGPCR, or functional equivalent, *in situ, i.e*., anchored in the cell membrane. Purification or enrichment of NGPCR from such expression systems can be accomplished using appropriate detergents and lipid micelles and methods well known to those skilled in the art. However, such engineered host cells themselves may be used in situations where it is important not only to retain the structural and functional characteristics of the NGPCR, but to assess biological activity, *e.g*., in drug screening assays.

The expression systems that may be used for purposes of the invention include but are not limited to microorganisms such as bacteria (*e.g., E. coli, B. subtilis*) transformed with recombinant bacteriophage DNA, plasmid DNA or cosmid DNA expression vectors containing NGPCR nucleotide sequences; yeast (*e.g*., *Saccharomyces, Pichia)* transformed with recombinant yeast expression vectors containing NGPCR nucleotide sequences; insect cell systems infected with recombinant virus expression vectors (*e.g*., baculovirus) containing NGPCR sequences; plant cell systems infected with recombinant virus expression vectors (*e.g.*, cauliflower mosaic virus, CaMV; tobacco mosaic virus, TMV) or transformed with recombinant plasmid expression vectors (*e.g.,* Ti plasmid) containing NGPCR nucleotide sequences; or mammalian cell systems (*e.g.*, COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In bacterial systems, a number of expression vectors may be advantageously selected depending upon the use intended for the NGPCR gene product being expressed. For example, when a large quantity of such a protein is to be produced, for the generation of pharmaceutical compositions of NGPCR protein or for raising antibodies to a NGPCR protein, for example, vectors that direct the expression of high levels of fusion protein products that are readily purified may be desirable. Such vectors include, but are not limited, to the *E. coli* expression vector pUR278 (Ruther et al., 1983, EMBO J. 2:1791), in which a NGPCR coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign polynucleotides. The virus grows in *Spodoptera frugiperda* cells. A NGPCR coding sequence may be cloned individually into non-essential regions (for example the polyhedrin gene) of the virus and placed under control of an AcNPV promoter (for example the polyhedrin promoter). Successful insertion of NGPCR coding sequence will result in inactivation of the polyhedrin gene and production of non-occluded recombinant virus (*i.e.*, virus lacking the proteinaceous coat coded for by the polyhedrin gene). These recombinant viruses are then used to infect Spodoptera frugiperda cells in which the inserted polynucleotide is expressed (*e.g.*, see Smith et al., 1983, J. Virol. 46: 584; Smith, U.S. Patent No. 4,215,051).

In mammalian host cells, a number of viral-based expression systems may be utilized. In cases where an adenovirus is used as an expression vector, the NGPCR nucleotide sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g.,* region E1 or E3) will result in a recombinant virus that is viable and capable of expressing a NGPCR gene product in infected hosts (*e.g*., See Logan & Shenk, 1984, Proc. Natl. Acad. Sci. USA 81:3655-3659). Specific initiation signals may also be required for efficient translation of inserted NGPCR nucleotide sequences. These signals include the ATG initiation codon and adjacent sequences. In cases where an entire NGPCR gene or cDNA, including its own initiation codon and adjacent sequences, is inserted into the appropriate expression vector, no additional translational control signals may be needed. However, in cases where only a portion of a NGPCR coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter et al., 1987, Methods in Enzymol. 153:516-544).

In addition, a host cell strain may be chosen that modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, and WI38 cell lines.

For long-term, high-yield production of recombinant proteins, stable expression is preferred. For example, cell lines which stably express the NGPCR sequences described above may be engineered. Rather than using expression vectors that contain viral origins of replication, host cells can be transformed with DNA controlled by appropriate expression control elements (*e.g.,* promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NGPCR gene product. Such engineered cell lines may be particularly useful in screening and evaluation of compounds that affect the endogenous activity of the NGPCR gene product.

A number of selection systems can be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147).

Alternatively, any fusion protein can be readily purified by utilizing an antibody specific for the fusion protein being expressed. For example, a system described by Janknecht *et al.* allows for the ready purification of non-denatured fusion proteins expressed in human cell lines (Janknecht, et al., 1991, Proc. Natl. Acad. Sci. USA 88: 8972-8976). In this system, the polynucleotide of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

NGPCR gene products can also be expressed in transgenic animals. Animals of any species, including, but not limited to, worms, mice, rats, rabbits, guinea pigs, pigs, micro-pigs, birds, goats, and non-human primates, *e.g*., baboons, monkeys, and chimpanzees may be used to generate NGPCR transgenic animals.

Any technique known in the art may be used to introduce a NGPCR transgene into animals to produce the founder lines of transgenic animals. Such techniques include, but are not limited to pronuclear microinjection (Hoppe, P.C. and Wagner, T.E., 1989, U.S. Pat. No. 4,873,191); retrovirus mediated gene transfer into germ lines (Van der Putten et al., 1985, Proc. Natl. Acad. Sci., USA 82:6148-6152); gene targeting in embryonic stem cells (Thompson et al., 1989, Cell 56:313-321); electroporation of embryos (Lo, 1983, Mol Cell. Biol. 3:1803-1814); and sperm-mediated gene transfer (Lavitrano et al., 1989, Cell 57:717-723); etc. For a review of such techniques, see Gordon, 1989, Transgenic Animals, Intl. Rev. Cytol. 115:171-229.

The present invention provides for transgenic animals that carry the NGPCR transgene in all their cells, as well as animals which carry the transgene in some, but not all their cells, *i.e.*, mosaic animals or somatic cell transgenic animals. The transgene may be integrated as a single transgene or in concatamers, *e.g*., head-to-head tandems or head-to-tail tandems. The transgene may also be selectively introduced into and activated in a particular cell type by following, for example, the teaching of Lasko et al., 1992, Proc. Natl. Acad. Sci. USA 89:6232-6236. The regulatory sequences required for such a cell-type specific activation will depend upon the particular cell type of interest, and will be apparent to those of skill in the art.

When it is desired that a NGPCR transgene be integrated into the chromosomal site of the endogenous NGPCR gene, gene targeting is preferred. Briefly, when such a technique is to be utilized, vectors containing some nucleotide sequences homologous to the endogenous NGPCR gene are designed for the purpose of integrating, via homologous recombination with chromosomal sequences, into and disrupting the function of the nucleotide sequence of the endogenous NGPCR gene (*i.e*., "knockout" animals).

The transgene can also be selectively introduced into a particular cell type, thus inactivating the endogenous NGPCR gene in only that cell type, by following, for example, the teaching of Gu et al., 1994, Science, 265:103-106. The regulatory sequences required for such a cell-type specific inactivation will depend upon the particular cell type of interest, and will be apparent to those of skill in the art.

Once transgenic animals have been generated, the expression of the recombinant NGPCR gene may be assayed utilizing standard techniques. Initial screening may be accomplished by Southern blot analysis or PCR techniques to analyze animal tissues to assay whether integration of the transgene has taken place. The level of mRNA expression of the transgene in the tissues of the transgenic animals may also be assessed using techniques which include but are not limited to Northern blot analysis of tissue samples obtained from the animal, *in situ* hybridization analysis, and RT-PCR. Samples of NGPCR gene-expressing tissue, may also be evaluated immunocytochemically using antibodies specific for the NGPCR transgene product.

Also encompassed by the present invention are fusion proteins that direct the NGPCR to a target organ and/or facilitate transport across the membrane into the cytosol. Conjugation of NGPCRs to antibody molecules or their Fab fragments could be used to target cells bearing a particular epitope. Attaching the appropriate signal sequence to the NGPCR would also transport the NGPCR to the desired location within the cell. Alternatively targeting of NGPCR or its nucleic acid sequence might be achieved using liposome or lipid complex based delivery systems. Such technologies are described in Liposomes:A Practical Approach, New, RRC ed., Oxford University Press, New York and in U.S. Patents Nos. 4,594,595, 5,459,127, 5,948,767 and 6,110,490 and their respective disclosures. Additionally embodied are novel protein constructs engineered in such a way that they facilitate transport of the NGPCR to the target site or desired organ, where they cross the cell membrane and/or the nucleus where the NGPCR can exert its functional activity. This goal may be achieved by coupling of the NGPCR to a cytokine or other ligand that provides targeting specificity, and/or to a protein transducing domain (see generally U.S. applications Ser. No. 60/111,701 and 60/056,713 for examples of such transducing sequences) to facilitate passage across cellular membranes and can optionally be engineered to include nuclear localization sequences.

### 5.3 ANTIBODIES TO NGPCR PROTEINS

Antibodies that specifically recognize one or more epitopes of a NGPCR, or epitopes of conserved variants of a NGPCR, or peptide fragments of a NGPCR are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NGPCR in a biological sample and may, therefore, be utilized as part of a diagnostic or prognostic technique whereby patients may be tested for abnormal amounts of NGPCR. Such antibodies may also be utilized in conjunction with, for example, compound screening schemes, as described below, for the evaluation of the effect of test compounds on expression and/or activity of a NGPCR gene product. Additionally, such antibodies can be used in conjunction gene therapy to, for example, evaluate the normal and/or engineered NGPCR-expressing cells prior to their introduction into the patient. Such antibodies may additionally be used as a method for the inhibition of abnormal NGPCR activity. Thus, such antibodies may, therefore, be utilized as part of weight disorder treatment methods.

For the production of antibodies, various host animals may be immunized by injection with the NGPCR, an NGPCR peptide (*e.g*., one corresponding to a functional domain of the receptor, such as an ECD, TM or CD), truncated NGPCR polypeptides (NGPCR in which one or more domains, *e.g*., a TM or CD, has been deleted), functional equivalents of the NGPCR or mutants of the NGPCR. Such host animals may include but are not limited to rabbits, mice, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures . Also encompassed by the present invention is the use of fully humanized monoclonal antibodies as described in US Patent No. 6,150,584 and respective disclosures.

Alternatively, techniques described for the production of single chain antibodies (U.S. Patent 4,946,778; Bird, 1988, Science 242:423-426; Huston et al., 1988, Proc. Natl. Acad. Sci. USA 85:5879-5883; and Ward et al., 1989, Nature 341:544-546) can be adapted to produce single chain antibodies against NGPCR gene products. Single chain antibodies are formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

Antibodies to a NGPCR can, in turn, be utilized to generate anti-idiotype antibodies that "mimic" a given NGPCR, using techniques well known to those skilled in the art. (See, *e.g.,* Greenspan & Bona, 1993, FASEB J 7(5):437-444; and Nissinoff, 1991, J. Immunol. 147(8):2429-2438). For example antibodies which bind to a NGPCR ECD and competitively inhibit the binding of a ligand of NGPCR can be used to generate anti-idiotypes that "mimic" a NGPCR ECD and, therefore, bind and neutralize a ligand. Such neutralizing anti-idiotypes or Fab fragments of such anti-idiotypes can be used in therapeutic regimens involving the NGPCR signaling pathway.

### 5.4 DIAGNOSIS OF ABNORMALITIES RELATED TO A NGPCR

A variety of methods can be employed for the diagnostic and prognostic evaluation of disorders related to NGPCR function, and for the identification of subjects having a predisposition to such disorders.

Such methods can, for example, utilize reagents such as the NGPCR nucleotide sequences described in Section 5.1, and NGPCR antibodies, as described, in Section 5.3. Specifically, such reagents may be used, for example, for: (1) the detection of the presence of NGPCR gene mutations, or the detection of either over- or under-expression of NGPCR mRNA relative to a given phenotype; (2) the detection of either an over- or an under-abundance of NGPCR gene product relative to a given phenotype; and (3) the detection of perturbations or abnormalities in the signal transduction pathway mediated by NGPCR.

The methods described herein may be performed, for example, by utilizing pre-packaged diagnostic kits comprising at least one specific NGPCR nucleotide sequence or NGPCR antibody reagent described herein, which may be conveniently used, *e.g*., in clinical settings, to diagnose patients exhibiting body weight disorder abnormalities.

For the detection of NGPCR mutations, any nucleated cell can be used as a starting source for genomic nucleic acid. For the detection of NGPCR gene expression or NGPCR gene products, any cell type or tissue in which the NGPCR gene is expressed, such as, for example, stomach or brain cells can be utilized.

Nucleic acid-based detection techniques and peptide detection techniques are described below.

### 5.4.1 DETECTION OF NGPCR GENES AND TRANSCRIPTS

Mutations within a NGPCR gene can be detected by utilizing a number of techniques. Nucleic acid from any nucleated cell can be used as the starting point for such assay techniques, and may be isolated according to standard nucleic acid preparation procedures which are well known to those of skill in the art.

DNA may be used in hybridization or amplification assays of biological samples to detect abnormalities involving NGPCR gene structure, including point mutations, insertions, deletions and chromosomal rearrangements. Such assays may include, but are not limited to, Southern analyses, single stranded conformational polymorphism analyses (SSCP), and PCR analyses.

Such diagnostic methods for the detection of NGPCR gene-specific mutations can involve for example, contacting and incubating nucleic acids including recombinant DNA molecules, cloned genes or degenerate variants thereof, obtained from a sample, *e.g*., derived from a patient sample or other appropriate cellular source, with one or more labeled nucleic acid reagents including recombinant DNA molecules, cloned genes or degenerate variants thereof, as described in Section 5.1, under conditions favorable for the specific annealing of these reagents to their complementary sequences within a given NGPCR gene. Preferably, the lengths of these nucleic acid reagents are at least 15 to 30 nucleotides. After incubation, all non-annealed nucleic acids are removed from the nucleic acid:NGPCR molecule hybrid. The presence of nucleic acids which have hybridized, if any such molecules exist, is then detected. Using such a detection scheme, the nucleic acid from the cell type or tissue of interest can be immobilized, for example, to a solid support such as a membrane, or a plastic surface such as that on a microtiter plate or polystyrene beads. In this case, after incubation, non-annealed, labeled nucleic acid reagents of the type described in Section 5.1 are easily removed. Detection of the remaining, annealed, labeled NGPCR nucleic acid reagents is accomplished using standard techniques well-known to those in the art. The NGPCR polynucleotide sequences to which the nucleic acid reagents have annealed can be compared to the annealing pattern expected from a normal NGPCR gene sequence in order to determine whether a NGPCR gene mutation is present.

Alternative diagnostic methods for the detection of NGPCR gene specific nucleic acid molecules, in patient samples or other appropriate cell sources, may involve their amplification, *e.g*., by PCR (the experimental embodiment set forth in Mullis, K.B., 1987, U.S. Patent No. 4,683,202), followed by the detection of the amplified molecules using techniques well known to those of skill in the art. The resulting amplified sequences can be compared to those which would be expected if the nucleic acid being amplified contained only normal copies of a NGPCR gene in order to determine whether a NGPCR gene mutation exists.

Additionally, well-known genotyping techniques can be performed to identify individuals carrying NGPCR gene mutations. Such techniques include, for example, the use of restriction fragment length polymorphisms (RFLPs), which involve sequence variations in one of the recognition sites for the specific restriction enzyme used.

Additionally, improved methods for analyzing DNA polymorphisms which can be utilized for the identification of NGPCR gene mutations'have been described which capitalize on the presence of variable numbers of short, tandemly repeated DNA sequences between the restriction enzyme sites. For example, Weber (U.S. Pat. No. 5,075,217 ) describes a DNA marker based on length polymorphisms in blocks of (dC-dA)n-(dG-dT)n short tandem repeats. The average separation of (dC-dA)n-(dG-dT)n blocks is estimated to be 30,000-60,000 bp. Markers which are so closely spaced exhibit a high frequency co-inheritance, and are extremely useful in the identification of genetic mutations, such as, for example, mutations within a given NGPCR gene, and the diagnosis of diseases and disorders related to NGPCR mutations.

Also, Caskey et al. (U.S. Pat. No. 5,364,759, which is incorporated herein by reference in its entirety) describe a DNA profiling assay for detecting short tri and tetra nucleotide repeat sequences. The process includes extracting the DNA of interest, such as the NGPCR gene, amplifying the extracted DNA, and labeling the repeat sequences to form a genotypic map of the individual's DNA.

The level of NGPCR gene expression can also be assayed by detecting and measuring NGPCR transcription. For example, RNA from a cell type or tissue known, or suspected to express the NGPCR gene, such as brain, may be isolated and tested utilizing hybridization or PCR techniques such as are described, above. The isolated cells can be derived from cell culture or from a patient. The analysis of cells taken from culture may be a necessary step in the assessment of cells to be used as part of a cell-based gene therapy technique or, alternatively, to test the effect of compounds on the expression of the NGPCR gene. Such analyses may reveal both quantitative and qualitative aspects of the expression pattern of the NGPCR gene, including activation or inactivation of NGPCR gene expression.

In one embodiment of such a detection scheme, cDNAs are synthesized from the RNAs of interest (*e.g*., by reverse transcription of the RNA molecule into cDNA). A sequence within the cDNA is then used as the template for a nucleic acid amplification reaction, such as a PCR amplification reaction, or the like. The nucleic acid reagents used as synthesis initiation reagents (*e.g*., primers) in the reverse transcription and nucleic acid amplification steps of this method are chosen from among the NGPCR nucleic acid reagents described in Section 5.1. The preferred lengths of such nucleic acid reagents are at least 9-30 nucleotides. For detection of the amplified product, the nucleic acid amplification may be performed using radioactively or non-radioactively labeled nucleotides. Alternatively, enough amplified product may be made such that the product may be visualized by standard ethidium bromide staining, by utilizing any other suitable nucleic acid staining method, or by sequencing.

Additionally, it is possible to perform such NGPCR gene expression assays "*in situ*", *i.e*., directly upon tissue sections (fixed and/or frozen) of patient tissue obtained from biopsies or resections, such that no nucleic acid purification is necessary. Nucleic acid reagents such as those described above may be used as probes and/or primers for such in situ procedures (See, for example, Nuovo, G.J., 1992, "PCR In Situ Hybridization: Protocols And Applications", Raven Press, NY).

Alternatively, if a sufficient quantity of the appropriate cells can be obtained, standard Northern analysis can be performed to determine the level of NGPCR mRNA expression.

### 5.4.2 DETECTION OF NGPCR GENE PRODUCTS

Antibodies directed against wild type or mutant NGPCR gene products or conserved variants or peptide fragments thereof, which are discussed above, may also be used as diagnostics and prognostics, as described herein. Such diagnostic methods, may be used to detect abnormalities in the level of NGPCR gene expression, or abnormalities in the structure and/or temporal, tissue, cellular, or subcellular location of the NGPCR, and may be performed *in vivo* or *in vitro*, such as, for example, on biopsy tissue.

For example, antibodies directed to epitopes of the NGPCR ECD can be used in vivo to detect the pattern and level of expression of the NGPCR in the body. Such antibodies can be labeled, *e.g*., with a radio-opaque or other appropriate compound and injected into a subject in order to visualize binding to the NGPCR expressed in the body using methods such as X-rays, CAT-scans, or MRI. Labeled antibody fragments, *e.g*., the Fab or single chain antibody comprising the smallest portion of the antigen binding region, are preferred for this purpose to promote crossing the blood-brain barrier and permit labeling NGPCRs expressed in the brain.

Additionally, any NGPCR fusion protein or NGPCR conjugated protein whose presence can be detected, can be administered. For example, NGPCR fusion or conjugated proteins labeled with a radio-opaque or other appropriate compound can be administered and visualized in vivo, as discussed, above for labeled antibodies. Further such NGPCR fusion proteins as AP-NGPCR on NGPCR-Ap fusion proteins can be utilized for *in vitro* diagnostic procedures.

Alternatively, immunoassays or fusion protein detection assays, as described above, can be utilized on biopsy and autopsy samples *in vitro* to permit assessment of the expression pattern of the NGPCR. Such assays are not confined to the use of antibodies that define a NGPCR ECD, but can include the use of antibodies directed to epitopes of any of the domains of a NGPCR, *e.g*., the ECD, the TM and/or CD. The use of each or all of these labeled antibodies will yield useful information regarding translation and intracellular transport of the NGPCR to the cell surface, and can identify defects in processing.

The tissue or cell type to be analyzed will generally include those which are known, or suspected, to express the NGPCR gene. The protein isolation methods employed herein may, for example, be such as those described in Harlow and Lane (Harlow, E. and Lane, D., 1988, "Antibodies: A Laboratory Manual", Cold Spring Harbor Laboratory Press, Cold Spring Harbor, New York) . The isolated cells can be derived from cell culture or from a patient. The analysis of cells taken from culture may be a necessary step in the assessment of cells that could be used as part of a cell-based gene therapy technique or, alternatively, to test the effect of compounds on the expression of a NGPCR gene.

For example, antibodies, or fragments of antibodies, such as those described, above, in Section 5.3, useful in the present invention may be used to quantitatively or qualitatively detect the presence of NGPCR gene products or conserved variants or peptide fragments thereof. This can be accomplished, for example, by immunofluorescence techniques employing a fluorescently labeled antibody (see below, this Section) coupled with light microscopic, flow cytometric, or fluorimetric detection. Such techniques are especially preferred if such NGPCR gene products are expressed on the cell surface.

The antibodies (or fragments thereof) or NGPCR fusion or conjugated proteins useful in the present invention may, additionally, be employed histologically, as in immunofluorescence, immunoelectron microscopy or non-immuno assays, for *in situ* detection of NGPCR gene products or conserved variants or peptide fragments thereof, or for NGPCR binding (in the case of labeled NGPCR ligand fusion protein).

*In situ* detection may be accomplished by removing a histological specimen from a patient, and applying thereto a labeled antibody or fusion protein of the present invention. The antibody (or fragment) or fusion protein is preferably applied by overlaying the labeled antibody (or fragment) onto a biological sample. Through the use of such a procedure, it is possible to determine not only the presence of a NGPCR gene product, or conserved variants or peptide fragments, or NGPCR binding, but also its distribution in the examined tissue. Using the present invention, those of ordinary skill will readily perceive that any of a wide variety of histological methods (such as staining procedures) can be modified in order to achieve such *in situ* detection.

Immunoassays and non-immunoassays for NGPCR gene products or conserved variants or peptide fragments thereof will typically comprise incubating a sample, such as a biological fluid, a tissue extract, freshly harvested cells, or lysates of cells which have been incubated in cell culture, in the presence of a detectably labeled antibody capable of identifying NGPCR gene products or conserved variants or peptide fragments thereof, and detecting the bound antibody by any of a number of techniques well-known in the art.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled NGPCR antibody or NGPCR ligand fusion protein. The solid phase support may then be washed with the buffer a second time to remove unbound antibody or fusion protein. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material can have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of NGPCR antibody or NGPCR ligand fusion protein may be determined according to well known methods. Those skilled in the art will be able to determine operative and optimal assay conditions for each determination by employing routine experimentation.

With respect to antibodies, one of the ways in which the NGPCR antibody can be detectably labeled is by linking the same to an enzyme and use in an enzyme immunoassay (EIA) (Voller, A., "The Enzyme Linked Immunosorbent Assay (ELISA)", 1978, Diagnostic Horizons 2:1-7, Microbiological Associates Quarterly Publication, Walkersville, MD); Voller, A. et al., 1978, J. Clin. Pathol. 31:507-520; Butler, J.E., 1981, Meth. Enzymol. 73:482-523; Maggio, E. (ed.), 1980, Enzyme Immunoassay, CRC Press, Boca Raton, FL,; Ishikawa, E. et al., (eds.), 1981, Enzyme Immunoassay, Kgaku Shoin, Tokyo). The enzyme that is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect NGPCR through the use of a radioimmunoassay (RIA) (see, for example, Weintraub, B., Principles of Radioimmunoassays, Seventh Training Course on Radioligand Assay Techniques, The Endocrine Society, March, 1986 ). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

### 5.5 SCREENING ASSAYS FOR COMPOUNDS THAT MODULATE NGPCR EXPRESSION OR ACTIVITY

The following assays are designed to identify compounds that interact with (*e.g*., bind to) NGPCRs (including, but not limited to an ECD or CD of a NGPCR), compounds that interact with (*e.g*., bind to) intracellular proteins that interact with NGPCR (including but not limited to the TM and CD of NGPCR), compounds that interfere with the interaction of NGPCR with transmembrane or intracellular proteins involved in NGPCR-mediated signal transduction, and to compounds which modulate the activity of NGPCR gene (*i.e*., modulate the level of NGPCR gene expression) or modulate the level of NGPCR. Assays may additionally be utilized which identify compounds which bind to NGPCR gene regulatory sequences (*e.g.*, promoter sequences) and which may modulate NGPCR gene expression. See *e.g*., Platt, K.A., 1994, J. Biol. Chem. 269:28558-28562.

The compounds that can be screened in accordance with the invention include but are not limited to peptides, antibodies and fragments thereof, and other organic compounds (*e.g.*, peptidomimetics) that bind to an ECD of a NGPCR and either mimic the activity triggered by the natural ligand (*i.e*., agonists) or inhibit the activity triggered by the natural ligand (*i.e*., antagonists); as well as peptides, antibodies or fragments thereof, and other organic compounds that mimic the ECD of the NGPCR (or a portion thereof) and bind to and "neutralize" the natural ligand.

Such compounds may include, but are not limited to, peptides such as, for example, soluble peptides, including but not limited to members of random peptide libraries; (see, *e.g*., Lam, K.S. et al., 1991, Nature 354:82-84; Houghten, R. et al., 1991, Nature 354:84-86), and combinatorial chemistry-derived molecular library made of D- and/or L- configuration amino acids, phosphopeptides (including, but not limited to members of random or partially degenerate, directed phosphopeptide libraries; see, *e.g.,* Songyang, Z. et al., 1993, Cell 72:767-778), antibodies (including, but not limited to, polyclonal, monoclonal, humanized, anti-idiotypic, chimeric or single chain antibodies, and FAb, F(ab')₂ and FAb expression library fragments, and epitope-binding fragments thereof), and small organic or inorganic molecules.

Other compounds which can be screened in accordance with the invention include but are not limited to small organic molecules that are able to cross the blood-brain barrier, gain entry into an appropriate cell (*e.g*., in the cerebellum, the hypothalamus, etc.) and affect the expression of a NGPCR gene or some other gene involved in the NGPCR signal transduction pathway (*e.g*., by interacting with the regulatory region or transcription factors involved in gene expression); or such compounds that affect the activity of the NGPCR (*e.g*., by inhibiting or enhancing the enzymatic activity of a CD) or the activity of some other intracellular factor involved in the NGPCR signal transduction pathway.

Computer modeling and searching technologies permit identification of compounds, or the improvement of already identified compounds, that can modulate NGPCR expression or activity. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential NGPCR modulating compounds.

Alternatively, these methods can be used to identify improved modulating compounds from an already known modulating compound or ligand. The composition of the known compound can be modified and the.structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active sites of a NGPCR, and related transduction and transcription factors will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen, et al., 1988, Acta Pharmaceutical Fennica 97:159-166; Ripka, New Scientist 54-57 (June 16, 1988); McKinaly and Rossmann, 1989, Annu. Rev. Pharmacol. Toxiciol. 29:111-122; Perry and Davies, OSAR: Quantitative Structure-Activity Relationships in Drug Design pp. 189-193 (Alan R. Liss, Inc. 1989); Lewis and Dean, 1989 Proc. R. Soc. Lond. 236:125-140 and 141-162; and, with respect to a model receptor for nucleic acid components, Askew, et al., 1989, J. Am. Chem. Soc. 111:1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

Although described above with reference to design and generation of compounds which could alter binding, one could also screen libraries of known compounds, including natural products or synthetic chemicals, and biologically active materials, including proteins, for compounds which are inhibitors or activators.

Cell-based systems can also be used to identify compounds that bind NGPCRs as well as assess the altered activity associated with such binding in living cells. One tool of particular interest for such assays is green fluorescent protein which is described, *inter alia*, in U.S. Patent No. 5, 625, 048. Cells that may be used in such cellular assays include, but are not limited to, leukocytes, or cell lines derived from leukocytes, lymphocytes, stem cells, including embryonic stem cells, and the like. In addition, expression host cells (*e.g*., B95 cells, COS cells, CHO cells, OMK cells, fibroblasts, Sf9 cells) genetically engineered to express a functional NGPCR of interest and to respond to activation by the test, or natural, ligand, as measured by a chemical or phenotypic change, or induction of another host cell gene, can be used as an end point in the assay.

Compounds identified via assays such as those described herein may be useful, for example, in elaborating the biological function of a NGPCR gene product. Such compounds can be administered to a patient at therapeutically effective doses to treat any of a variety of physiological or mental disorders. A therapeutically effective dose refers to that amount of the compound sufficient to result in any amelioration, impediment, prevention, or alteration of any biological or overt symptom.

Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients. Thus, the compounds and their physiologically acceptable salts and solvates may be formulated for administration by inhalation or insufflation (either through the mouth or the nose) or oral, buccal, parenteral, intracranial, intrathecal, or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g.*, sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g.*, almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g*. gelatin for use in an inhaler or insufflator may be formulated containing a powder mix of the compound and a suitable powder base such as lactose or starch.

The compounds may be formulated for parenteral administration by injection, *e.g.*, by bolus injection or continuous infusion. Formulations for injection may be presented in unit dosage form, *e.g*., in ampoules or in multidose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The compounds may also be formulated in rectal compositions such as suppositories or retention enemas, *e.g*., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 5.5.1 IN VITRO SCREENING ASSAYS FOR COMPOUNDS THAT BIND TO NGPCRs

*In vitro* systems can be designed to identify compounds capable of interacting with (*e.g.*, binding to) NGPCR (including, but not limited to, a ECD or CD of NGPCR). Compounds identified may be useful, for example, in modulating the activity of wild type and/or mutant NGPCR gene products; may be useful in elaborating the biological function of the NGPCR; may be utilized in screens for identifying compounds that disrupt normal NGPCR interactions; or may in themselves disrupt such interactions.

The principle of the assays used to identify compounds that bind to the NGPCR involves preparing a reaction mixture of the NGPCR and the test compound under conditions and for a time sufficient to allow the two components to interact and bind, thus forming a complex which can be removed and/or detected in the reaction mixture. The NGPCR species used can vary depending upon the goal of the screening assay. For example, where agonists of the natural ligand are sought, the full length NGPCR, or a soluble truncated NGPCR, *e.g*., in which the TM and/or CD is deleted from the molecule, a peptide corresponding to a ECD or a fusion protein containing one or more NGPCR ECD fused to a protein or polypeptide that affords advantages in the assay system (*e.g*., labeling, isolation of the resulting complex, etc.) can be utilized. Where compounds that interact with the cytoplasmic domain are sought to be identified, peptides corresponding to the NGPCR CD and fusion proteins containing the NGPCR CD can be used.

The screening assays can be conducted in a variety of ways. For example, one method to conduct such an assay would involve anchoring the NGPCR protein, polypeptide, peptide or fusion protein or the test substance onto a solid phase and detecting NGPCR/test compound complexes anchored on the solid phase at the end of the reaction. In one embodiment of such a method, the NGPCR reactant may be anchored onto a solid surface, and the test compound, which is not anchored, may be labeled, either directly or indirectly.

In practice, microtiter plates may conveniently be utilized as the solid phase. The anchored component may be immobilized by non-covalent or covalent attachments. Non-covalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the nonimmobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously nonimmobilized component is pre-labeled, the detection of label immobilized on the surface indicates that complexes were formed. Where the previously nonimmobilized component is not pre-labeled, an indirect label can be used to detect complexes anchored on the surface; *e.g*., using a labeled antibody specific for the previously nonimmobilized component (the antibody, in turn, may be directly labeled or indirectly labeled with a labeled anti-Ig antibody).

Alternatively, a reaction can be conducted in a liquid phase, the reaction products separated from unreacted components, and complexes detected; *e.g*., using an immobilized antibody specific for a NGPCR protein, polypeptide, peptide or fusion protein or the test compound to anchor any complexes formed in solution, and a labeled antibody specific for the other component of the possible complex to detect anchored complexes.

Alternatively, cell-based.assays can be used to identify compounds that interact with NGPCR. To this end, cell lines that express NGPCR, or cell lines (*e.g*., COS cells, CHO cells, fibroblasts, etc.) that have been genetically engineered to express a NGPCR (*e.g*., by transfection or transduction of NGPCR DNA) can be used. Interaction of the test compound with, for example, a ECD of a NGPCR expressed by the host cell can be determined by comparison or competition with native ligand.

### 5.5.2. ASSAYS FOR INTRACELLULAR PROTEINS THAT INTERACT WITH NGPCRs

Any method suitable for detecting protein-protein interactions may be employed for identifying transmembrane proteins or intracellular proteins that interact with a NGPCR. Among the traditional methods which may be employed are co-immunoprecipitation, crosslinking and co-purification through gradients or chromatographic columns of cell lysates or proteins obtained from cell lysates and a NGPCR to identify proteins in the lysate that interact with the NGPCR. For these assays, the NGPCR component used can be a full length NGPCR, a soluble derivative lacking the membrane-anchoring region (*e.g*., a truncated NGPCR in which a TM is deleted resulting in a truncated molecule containing a ECD fused to a CD), a peptide corresponding to a CD or a fusion protein containing a CD of a NGPCR. Once isolated, such an intracellular protein can be identified and can, in turn, be used, in conjunction with standard techniques, to identify proteins with which it interacts. For example, at least a portion of the amino acid sequence of an intracellular protein which interacts with a NGPCR can be ascertained using techniques well known to those of skill in the art, such as via the Edman degradation technique. (See, *e.g*., Creighton, 1983, "Proteins: Structures and Molecular Principles", W.H. Freeman & Co., N.Y., pp.34-49). The amino acid sequence obtained may be used as a guide for the generation of oligonucleotide mixtures that can be used to screen for gene sequences encoding such intracellular proteins. Screening can be accomplished, for example, by standard hybridization or PCR techniques. Techniques for the generation of oligonucleotide mixtures and the screening are well-known. (See, *e.g*., Ausubel, supra, and PCR Protocols: A Guide to Methods and Applications, 1990, Innis, M. et al., eds. Academic Press, Inc., New York).

Additionally, methods may be employed which result in the simultaneous identification of genes which encode the transmembrane or intracellular proteins interacting with NGPCR. These methods include, for example, probing expression, libraries, in a manner similar to the well known technique of antibody probing of λgt11 libraries, using labeled NGPCR protein, or an NGPCR polypeptide, peptide or fusion protein, *e.g*., an NGPCR polypeptide or NGPCR domain fused to a marker (*e.g*., an enzyme, fluor, luminescent protein, or dye), or an Ig-Fc domain.

One method that detects protein interactions *in vivo,* the two-hybrid system, is described in detail for illustration only and not by way of limitation. One version of this system has been described (Chien et al., 1991, Proc. Natl. Acad. Sci. USA, 88:9578-9582) and is commercially available from Clontech (Palo Alto, CA).

Briefly, utilizing such a system, plasmids are constructed that encode two hybrid proteins: one plasmid consists of nucleotides encoding the DNA-binding domain of a transcription activator protein fused to a NGPCR nucleotide sequence encoding NGPCR, an NGPCR polypeptide, peptide or fusion protein, and the other plasmid consists of nucleotides encoding the transcription activator protein's activation domain fused to a cDNA encoding an unknown protein which has been recombined into this plasmid as part of a cDNA library. The DNA-binding domain fusion plasmid and the cDNA library are transformed into a strain of the yeast *Saccharomyces cerevisiae* that contains a reporter gene (*e.g.,* HBS or *lacZ*) whose regulatory region contains the transcription activator's binding site. Either hybrid protein alone cannot activate transcription of the reporter gene: the DNA-binding domain hybrid cannot because it does not provide activation function and the activation domain hybrid cannot because it cannot localize to the activator's binding sites. Interaction of the two hybrid proteins reconstitutes the functional activator protein and results in expression of the reporter gene, which is detected by an assay for the reporter gene product.

The two-hybrid system or related methodology may be used to screen activation domain libraries for proteins that interact with the "bait" gene product. By way of example, and not by way of limitation, a NGPCR may be used as the bait gene product. Total genomic or cDNA sequences are fused to the DNA encoding an activation domain. This library and a plasmid encoding a hybrid of a bait NGPCR gene product fused to the DNA-binding domain are cotransformed into a yeast reporter strain, and the resulting transformants are screened for those that express the reporter gene. For example, and not by way of limitation, a bait NGPCR gene sequence, such as the open reading frame of a NGPCR (or a domain of a NGPCR) can be cloned into a vector such that it is translationally fused to the DNA encoding the DNA-binding domain of the GAL4 protein. These colonies are purified and the library plasmids responsible for reporter gene expression are isolated. DNA sequencing is then used to identify the proteins encoded by the library plasmids.

A cDNA library of the cell line from which proteins that interact with bait NGPCR gene product are to be detected can be made using methods routinely practiced in the art. According to the particular system described herein, for example, the cDNA fragments can be inserted into a vector such that they are translationally fused to the transcriptional activation domain of GAL4. This library can be co-transformed along with the bait NGPCR gene-GAL4 fusion plasmid into a yeast strain which contains a lacZ gene driven by a promoter which contains GAL4 activation sequence. A cDNA encoded protein, fused to GAL4 transcriptional activation domain, that interacts with bait NGPCR gene product will reconstitute an active GAL4 protein and thereby drive expression of the HIS3 gene. Colonies which express HIS3 can be detected by their growth on petri dishes containing semi-solid agar based media lacking histidine. The cDNA can then be purified from these strains, and used to produce and isolate the bait NGPCR gene-interacting protein using techniques routinely practiced in the art.

### 5.5.3. ASSAYS FOR COMPOUNDS THAT INTERFERE WITH NGPCR/INTRACELLULAR OR NGPCR/TRANSMEMBRANE MACROMOLECULE INTERACTION

The macromolecules that interact with the NGPCR are referred to, for purposes of this discussion, as "binding partners." These binding partners are likely to be involved in the NGPCR signal transduction pathway. Therefore, it is desirable to identify compounds that interfere with or disrupt the interaction of such binding partners which may be useful in regulating the activity of a NGPCR and controlling disorders associated with NGPCR activity. For example, given their expression pattern, the described NGPCRs are contemplated to be particularly useful in methods for identifying compounds useful in the therapeutic treatment of obesity, inflammation, immune disorders, diabetes, heart and coronary disease, metabolic disorders, and cancer.

The basic principle of the assay systems used to identify compounds that interfere with the interaction between a NGPCR and its binding partner or partners involves preparing a reaction mixture containing NGPCR protein, polypeptide, peptide or fusion protein as described in Sections 5.5.1 and 5.5.2 above, and the binding partner under conditions and for a time sufficient to allow the two to interact and bind, thus forming a complex. In order to test a compound for inhibitory activity, the reaction mixture is prepared in the presence and absence of the test compound. The test compound may be initially included in the reaction mixture, or may be added at a time subsequent to the addition of the NGPCR moiety and its binding partner. Control reaction mixtures are incubated without the test compound or with a placebo. The formation of any complexes between the NGPCR moiety and the binding partner is then detected. The formation of a complex in the control reaction, but not in the reaction mixture containing the test compound, indicates that the compound interferes with the interaction of the NGPCR and the interactive binding partner. Additionally, complex formation within reaction mixtures containing the test compound and normal NGPCR protein may also be compared to complex formation within reaction mixtures containing the test compound and a mutant NGPCR. This comparison may be important in those cases wherein it is desirable to identify compounds that specifically disrupt interactions of mutant, or mutated, NGPCRs but not normal NGPCRs.

The assay for compounds that interfere with the interaction of a NGPCR and its binding partners can be conducted in a heterogeneous or homogeneous format. Heterogeneous assays involve anchoring either the NGPCR moiety product or the binding partner onto a solid phase and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction by competition can be identified by conducting the reaction in the presence of the test substance; *i.e.*, by adding the test substance to the reaction mixture prior to, or simultaneously with, a NGPCR moiety and interactive binding partner. Alternatively, test compounds that disrupt preformed complexes, *e.g*. compounds with higher binding constants that displace one of the components from the complex, can be tested by adding the test compound to the reaction mixture after complexes have been formed. The various formats are described briefly below.

In a heterogeneous assay system, either a NGPCR moiety or an interactive binding partner, is anchored onto a solid surface, while the non-anchored species is labeled, either directly or indirectly. In practice, microtiter plates are conveniently utilized. The anchored species may be immobilized by non-covalent or covalent attachments. Non-covalent attachment may be accomplished simply by coating the solid surface with a solution of the NGPCR gene product or binding partner and drying. Alternatively, an immobilized antibody specific for the species to be anchored may be used to anchor the species to the solid surface. The surfaces may be prepared in advance and stored.

In order to conduct the assay, the partner of the immobilized species is exposed to the coated surface with or without the test compound. After the reaction is complete, unreacted components are removed (*e.g.*, by washing) and any complexes formed will remain immobilized on the solid surface. The detection of complexes anchored on the solid surface can be accomplished in a number of ways. Where the non-immobilized species is pre-labeled, the detection of label immobilized on the surface indicates that complexes were formed. Where the non-immobilized species is not pre-labeled, an indirect label can be used to detect complexes anchored on the surface; *e.g.*, using a labeled antibody specific for the initially non-immobilized species (the antibody, in turn, may be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test compound, the reaction products separated from unreacted components, and complexes detected; *e.g*., using an immobilized antibody specific for one of the binding components to anchor any complexes formed in solution, and a labeled antibody specific for the other partner to detect anchored complexes. Again, depending upon the order of addition of reactants to the liquid phase, test compounds which inhibit complex or which disrupt preformed complexes can be identified.

In an alternate embodiment of the invention, a homogeneous assay can be used. In this approach, a preformed complex of a NGPCR moiety and an interactive binding partner is prepared in which either the NGPCR or its binding partners is labeled, but the signal generated by the label is quenched due to formation of the complex (see, *e.g*., U.S. Patent No. 4,109,496 by Rubenstein which utilizes this approach for immunoassays). The addition of a test substance that competes with and displaces one of the species from the preformed complex will result in the generation of a signal above background. In this way, test substances which disrupt NGPCR/intracellular binding partner interaction can be identified.

In a particular embodiment, a NGPCR fusion can be prepared for immobilization. For example, a NGPCR or a peptide fragment, *e.g.*, corresponding to a CD, can be fused to a glutathione-S-transferase (GST) gene using a fusion vector, such as pGEX-5X-1, in such a manner that its binding activity is maintained in the resulting fusion protein. The interactive binding partner can be purified and used to raise a monoclonal antibody, using methods routinely practiced in the art and described above, in Section 5.3. This antibody can be labeled with the radioactive isotope ¹²⁵I, for example, by methods routinely practiced in the art. In a heterogeneous assay, *e.g.*, the GST-NGPCR fusion protein can be anchored to glutathione-agarose beads. The interactive binding partner can then be added in the presence or absence of the test compound in a manner that allows interaction and binding to occur. At the end of the reaction period, unbound material can be washed away, and the labeled monoclonal antibody can be added to the system and allowed to bind to the complexed components. The interaction between a NGPCR gene product and the interactive binding partner can be detected by measuring the amount of radioactivity that remains associated with the glutathione-agarose beads. A successful inhibition of the interaction by the test compound will result in a decrease in measured radioactivity.

Alternatively, the GST-NGPCR fusion protein and the interactive binding partner can be mixed together in liquid in the absence of the solid glutathione-agarose beads. The test compound can be added either during or after the species are allowed to interact. This mixture can then be added to the glutathione-agarose beads and unbound material is washed away. Again the extent of inhibition of the NGPCR/binding partner interaction can be detected by adding the labeled antibody and measuring the radioactivity associated with the beads.

In another embodiment of the invention, these same techniques can be employed using peptide fragments that correspond to the binding domains of a NGPCR and/or the interactive or binding partner (in cases where the binding partner is a protein), in place of one or both of the full length proteins. Any number of methods routinely practiced in the art can be used to identify and isolate the binding sites. These methods include, but are not limited to, mutagenesis of the gene encoding one of the proteins and screening for disruption of binding in a co-immunoprecipitation assay. Compensatory mutations in the gene encoding the second species in the complex can then be selected. Sequence analysis of the genes encoding the respective proteins will reveal the mutations that correspond to the region of the protein involved in interactive binding. Alternatively, one protein can be anchored to a solid surface using methods described above, and allowed to interact with and bind to its labeled binding partner, which has been treated with a proteolytic enzyme, such as trypsin. After washing, a relatively short, labeled peptide comprising the binding domain may remain associated with the solid material, which can be isolated and identified by amino acid sequencing. Also, once the gene coding for the intracellular binding partner is obtained, short gene segments can be engineered to express peptide fragments of the protein, which can then be tested for binding activity and purified or synthesized.

For example, and not by way of limitation, a NGPCR gene product can be anchored to a solid material as described, above, by making a GST-NGPCR fusion protein and allowing it to bind to glutathione agarose beads. The interactive binding partner can be labeled with a radioactive isotope, such as ³⁵S, and cleaved with a proteolytic enzyme such as trypsin. Cleavage products can then be added to the anchored GST-NGPCR fusion protein and allowed to bind. After washing away unbound peptides, labeled bound material, representing the intracellular binding partner binding domain, can be eluted, purified, and analyzed for amino acid sequence by well-known methods. Peptides so identified can be produced synthetically or fused to appropriate facilitative proteins using recombinant DNA technology.

## Claims

1. Use of a nucleic acid molecule comprising a nucleotide sequence that encodes the amino acid sequence shown in SEQ ID NO: 2, SEQ ID NO: 4 or SEQ ID NO: 6 in a method of diagnosing a weight disorder, a method of screening drugs for use in the treatment of a weight disorder or a method of monitoring a clinical trial of a treatment for a weight disorder, subject to the proviso that methods for treatment of the human or animal body by surgery or therapy and diagnostic methods practised on the human or animal body are not claimed.

2. Use of a polypeptide comprising the amino acid sequence shown in SEQ ID NO: 2, SEQ ID NO: 4 or SEQ ID NO: 6 in a method of diagnosing a weight disorder, a method of screening drugs for use in the treatment of a weight disorder or a method of monitoring a clinical trial of a treatment for a weight disorder, subject to the proviso that methods for treatment of the human or animal body by surgery or therapy and diagnostic methods practised on the human or animal body are not claimed.

3. Use of an antibody that specifically recognises one or more epitopes of a polypeptide comprising the amino acid sequence shown in SEQ ID NO: 2, SEQ ID NO: 4 or SEQ ID NO: 6, or an epitope-binding fragment or derivative of said antibody, for example, a polyclonal or a monoclonal antibody, a single chain antibody, a Fab fragment, a F(ab')₂, a fragment produced by a Fab expression library, or an anti-idiotypic (anti-Id) antibody, in a method of diagnosing a weight disorder, a method of screening drugs for use in the treatment of a weight disorder or a method of monitoring a clinical trial of a treatment for a weight disorder, subject to the proviso that methods for treatment of the human or animal body by surgery or therapy and diagnostic methods practised on the human or animal body are not claimed.

4. Use of an antibody that specifically recognises one or more epitopes of a polypeptide comprising the amino acid sequence shown in SEQ ID NO: 2, SEQ ID NO: 4 or SEQ ID NO: 6, or an epitope-binding fragment or derivative of said antibody, for example, a polyclonal or a monoclonal antibody, a single chain antibody, a Fab fragment, a F(ab')₂, a fragment produced by a Fab expression library, or an anti-idiotypic (anti-Id) antibody, in the manufacture of a medicament for the treatment of a weight disorder.

5. An antisense nucleic acid molecule that inhibits the expression of a polypeptide comprising the amino acid sequence shown in SEQ ID NO: 2, SEQ ID NO: 4 or SEQ ID NO: 6 for use as a medicament for the treatment of a weight disorder.

6. Use of an antisense nucleic acid molecule that inhibits expression of a polypeptide comprising the amino acid sequence shown in SEQ ID NO: 2, SEQ ID NO: 4 or SEQ ID NO: 6 in the manufacture of a medicament for the treatment of a weight disorder.

## Patentansprüche

1. Verwendung eines Nukleinsäuremoleküls umfassend eine Nucleotidsequenz, welche für die Aminosäuresequenz, die in SEQ ID NO: 2, SEQ ID NO: 4 oder SEQ ID NO: 6 dargestellt wird, kodiert, in einem Verfahren zur Diagnose einer Gewichtsstörung, einem Verfahren zum Wirkstoffscreening zur Verwendung bei der Behandlung einer Gewichtsstörung oder einem Verfahren zur Überwachung eines klinischen Versuchs einer Behandlung einer Gewichtsstörung, unter der Bedingung, dass Verfahren zur chirurgischen oder therapeutischen Behandlung des menschlichen oder tierischen Körpers und Diagnostizierverfahren, die am menschlichen oder tierischen Körper vorgenommen werden, nicht beansprucht sind.

2. Verwendung eines Polypeptids umfassend die Aminosäuresequenz, die in SEQ.ID NO: 2, SEQ ID NO: 4 oder SEQ ID NO: 6 dargestellt wird, in einem Verfahren zur Diagnose einer Gewichtsstörung, in einem Verfahren zum Wirkstoffscreening zur Verwendung bei der Behandlung einer Gewichtsstörung oder in einem Verfahren zur Überwachung eines klinischen Versuchs einer Behandlung einer Gewichtsstörung, mit der Einschränkung, dass Verfahren zur chirurgischen oder therapeutischen Behandlung des menschlichen oder tierischen Körpers und Diagnostizierverfahren, die am menschlichen oder tierischen Körper vorgenommen werden, nicht beansprucht sind.

3. Verwendung eines Antikörpers, welcher spezifisch ein oder mehrere Epitope eines Polypeptids erkennt, welches die Aminosäuresequenz umfasst, die in SEQ ID NO: 2, SEQ ID NO: 4 oder SEQ ID NO: 6 dargestellt ist, oder eines Epitop bindenden Fragments oder Derivats des Antikörpers, z. B., eines polyklonalen oder monoklonalen Antikörpers, eines einsträngigen Antikörpers, eines Fab Fragments, eines F (ab')₂, eines Fragments hergesellt mittels einer Fab Expressionsbibliothek, oder eines anti - idiotypischen (anti Id) Antikörpers, in einem Verfahren zur Diagnose einer Gewichtsstörung, einem Verfahren zum Wirkstoffscrenning zur Verwendung bei der Behandlung einer Gewichtsstörung oder in einem Verfahren zur Überwachung eines klinischen Versuchs einer Behandlung einer Gewichtsstörung, mit der Einschränkung, dass Verfahren zur chirurgischen oder therapeutischen Behandlung des menschlichen oder tierischen Körpers und Diagnostizierverfahren, die am menschlichen oder tierischen Körper vorgenommen werden, nicht beansprucht sind.

4. Verwendung eines Antikörpers, welcher spezifisch ein oder mehrere Epitope eines Polypeptids erkennt, welches die Aminosäuresequenz, welche in SEQ ID NO: 2, SEQ ID NO: 4, SEQ ID NO: 6 dargestellt ist umfasst, oder eines Epitop bindenden Fragments oder Derivats des Antikörpers, beispielsweise eines polyklonalen oder eines monoklonalen Antikörpers, eines einsträngigen Antikörpers, eines Fab Fragments, eines F (ab')₂, eines Fragments hergestellt mittels einer Fab Expressionsbibliothek, oder eines anti - idiotypischen (anti - Id) Antikörpers, bei der Herstellung eines Medikaments zur Behandlung einer Gewichtsstörung.

5. Ein Antisens - Nukleinsäuremolekül, welches die Expression eines Polypeptids inhibiert, umfassend die Aminosäuresequenz, welche in SEQ ID NO: 2, SEQ ID NO: 4 oder SEQ ID NO: 6 dargestellt wird, zur Verwendung als Medikament zur Behandlung einer Gewichtsstörung.

6. Verwendung eines Antisens - Nukleinsäuremoleküls, welches die Expression eines Polypeptids inhibiert, das die Aminosäuresequenz umfasst, die in SEQ ID NO: 2, SEQ ID NO: 4 oder SEQ ID NO: 6 dargestellt wird bei, der Herstellung eines Medikaments zur Behandlung einer Gewichtsstörung.

## Revendications

1. Utilisation d'une molécule d'acide nucléique comprenant une séquence nucléotidique qui code pour la séquence d'acides aminés représentée dans SEQ ID NO. 2, SEQ ID NO. 4 ou SEQ ID NO. 6 dans un procédé de diagnostic d'un trouble pondéral, un procédé de criblage de médicaments destinés à être utilisés dans le traitement d'un trouble pondéral, ou un procédé de monitorage d'un essai clinique d'un traitement destiné à un trouble pondéral, sous réserve que les procédés de traitement de l'organisme humain ou animal par chirurgie et thérapie et les procédés de diagnostic pratiqués sur l'organisme humain ou l'animal ne soient pas revendiqués.

2. Utilisation d'un polypeptide comprenant la séquence d'acides aminés représentée dans SEQ ID NO. 2, SEQ ID NO. 4 ou SEQ ID NO. 6 dans un procédé de diagnostic d'un trouble pondéral, un procédé de criblage de médicaments destinés à être utilisés dans le traitement d'un trouble pondéral ou un procédé de monitorage d'un essai clinique d'un traitement destiné à un trouble pondéral, sous réserve que les procédés de traitement de l'organisme humain ou animal par chirurgie et thérapie et les procédés de diagnostic pratiqués sur l'organisme humain ou l'animal ne soient pas revendiqués.

3. Utilisation d'un anticorps qui reconnaît spécifiquement un ou plusieurs épitopes d'un polypeptide comprenant la séquence d'acides aminés représentée dans SEQ ID NO. 2, SEQ ID NO. 4 ou SEQ ID NO. 6, ou d'un fragment de liaison aux épitopes ou dérivé dudit anticorps, par exemple un anticorps polyclonal ou monoclonal, un anticorps simple chaîne, un fragment Fab, F(ab')₂, un fragment produit par une banque d'expression de Fab, ou un anticorps anti-idiotypique (anti-Id), dans un procédé de diagnostic d'un trouble pondéral, un procédé de criblage de médicaments destinés à être utilisés dans le traitement destiné à un trouble pondéral ou un procédé de monitorage d'un essai clinique d'un traitement destiné à un trouble pondéral, sous réserve que les procédés de traitement de l'organisme humain ou animal par chirurgie et thérapie et les procédés de diagnostic pratiqués sur l'organisme humain ou l'animal ne soient pas revendiqués.

4. Utilisation d'un anticorps qui reconnaît spécifiquement un ou plusieurs épitopes d'un polypeptide comprenant la séquence d'acides aminés représentée dans SEQ ID NO. 2, SEQ ID NO. 4 ou SEQ ID NO. 6, ou d'un fragment de liaison aux épitopes ou dérivé dudit anticorps, par exemple un anticorps polyclonal ou monoclonal, un anticorps simple chaîne, un fragment Fab, F(ab')₂, un fragment produit par une banque d'expression de Fab, ou un anticorps anti-idiotypique (anti-Id), dans la fabrication d'un médicament destiné au traitement d'un trouble pondéral.

5. Molécule d'acide nucléique antisens qui inhibe l'expression d'un polypeptide comprenant la séquence d'acides aminés représentée dans SEQ ID NO. 2, SEQ ID NO. 4 ou SEQ ID NO. 6, pour une utilisation comme médicament destiné au traitement d'un trouble pondéral.

6. Utilisation d'une molécule d'acide nucléique antisens qui inhibe l'expression d'un polypeptide comprenant la séquence d'acides aminés représentée dans SEQ ID NO. 2, SEQ ID NO. 4 ou SEQ ID NO. 6 dans la fabrication d'un médicament destiné au traitement d'un trouble pondéral.


### SEQUENCE LISTING

<110> LEXICON GENETICS INCORPORATED
<120> Novel Human 7TM Proteins and Polynucleotides Encoding the Same
<130> LEX-0157-PCT
<150> US 60/192,978
   <151> 2000-03-28
<160> 9
<170> FastSEQ for Windows Version 4.0
<210> 1
   <211> 2526
   <212> DNA
   <213> homo sapiens
<400> 1
   atgctgctct gcacggctcg cctggtcggc ctgcagcttc tcatttcctg ctgctgggcc      60
   tttgcctgcc atagcacgga gtcttctcct gacttcaccc tccccggaga ttacctcctg     120
   gcaggcctgt tccctctcca ttctggctgt ctgcaggtga ggcacagacc cgaggtgacc     180
   ctgtgtgaca ggtcttgtag cttcaatgag catggctacc acctcttcca ggctatgcgg     240
   cttggggttg aggagataaa caactccacg gccctgctgc ccaacatcac cctggggtac     300
   cagctgtatg atgtgtgtty tgactctgcc aatgtgtatg ccacgctgag agtgctctcc     360
   ctgccagggc aacaccacat agagctccaa ggagaccttc tccactattc ccctacggtg     420
   ctggcagtga ttgggcctga cagcaccaac cgtgctgcca ccacagccgc cctgctgagc     480
   cctttcctgg tgcccatgat tagctatgcg gccagcagcg agacgctcag cgtgaagcgg     540
   cagtatccct ctttcctgcg caccatcccc aatgacaagt accaggtgga gaccatggtg     600
   ctgctgctgc agaagttcgg gtggacctgg atctctctgg ttggcagcag tgacgactat     660
   gggcagctag gggtgcaggc actggagaac caggccactg gtcaggggat ctgcattgct     720
   ttcaaggaca tcatgccctt ctctgcccag gtgggcgatg agaggatgca gtgcctcatg     780
   cgccacctgg cccaggccgg ggccaccgtc gtggttgttt tttccagccg gcagttggcc     840
   agggtgtttt tcgagtccgt ggtgctgacc aacctgactg gcaaggtgtg ggtcgcctca     900
   gaagcctggg ccctctccag gcacatcact ggggtgcccg ggatccagcg cattgggatg     960
   gtgctgggcg tggccatcca gaagagggct gtccctggcc tgaaggcgtt tgaagaagcc    1020
   tatgcccggg cagacaagga ggcccctagg ccttgccaca agggctcctg gtgcagcagc    1080
   aatcagctct gcagagaatg ccaagctttc atgrcacaca cgatgcccaa gctcaaagcc    1140
   ttctccatga gttctgccta caacgcatac cgggctgtgt atgcggtggc ccatggcctc    1200
   caccagctcc tgggctgtgc ctctggagct tgttccaggg gccgagtcta cccctggcag    1260
   cttttggagc agatccacaa ggtgcatttc cttctacaca aggacactgt ggcgtttaat    1320
   gacaacagag atcccctcag tagctataac ataattgcct gggactggaa tggacccaag    1380
   tggaccttca cggtcctcgg ttcctccaca tggtctccag ttcagctaaa cataaatgag    1440
   accaaaatcc agtggcacgg aaaggacaac caggtgccta agtctgtgtg ttccagcgac    1500
   tgtcttgaag ggcaccagcg agtggttacg ggtttccatc actgctgctt tgagtgtgtg    1560
   ccctgtgggg ctgggacctt cctcaacaag agtgacctct acagatgcca gccttgtggg    1620
   aaagaagagt gggcacctga gggaagccag acctgcttcc cgcgcactgt ggtgtttttg    1680
   gctttgcgtg agcacacctc ttgggtgctg ctggcagcta acacgctgct gctgctgctg    1740
   ctgcttggga ctgctggcct gtttgcctgg cacctagaca cccctgtggt gaggtcagca    1800
   gggggccgcc tgtgctttct tatgctgggc tccctggcag caggtagtgg cagcctctat    1860
   ggcttctttg gggaacccac aaggcctgcg tgcttgctac gccaggccct ctttgccctt    1920
   ggtttcacca tcttcctgtc ctgcctgaca gttcgctcat tccaactaat catcatcttc    1980
   aagttttcca ccaaggtacc tacattctac cacgcctggg tccaaaacca cggtgctggc    2040
   ctgtttgtga tgatcagctc agcggcccag ctgcttatct gtctaacttg gctggtggtg    2100
   tggaccccac tgcctgctag ggaataccag cgcttccccc atctggtgat gcttgagtgc    2160
   acagagacca actccctggg cttcatactg gccttcctct acaatggcct cctctccatc    2220
   agtgcctttg cctgcagcta cctgggtaag gacttgccag agaactacaa cgaggccaaa    2280
   tgygtcacct tcagcctgct cttcaacttc gtgtcctgga tcgccttctt caccacggcc    2340
   agcgtctacg acggcaagta cctgcctgcg gccaacatga tggctgggct gagcagcctg    2400
   agcagcggct tcggtgggta ttttctgcct aagtgctacg tgatcctctg ccgcccagac    2460
   ctcaacagca cagagcactt ccaggcctcc attcaggact acacgaggcg ctgcggctcc    2520
   acctga                                                              2526
<210> 2
   <211> 841
   <212> PRT
   <213> homo sapiens
<400> 2
<210> 3
   <211> 2292
   <212> DNA
   <213> homo sapiens
<400> 3
   atgcggcttg gggttgagga gataaacaac tccacggccc tgctgcccaa catcaccctg      60
   gggtaccagc tgtatgatgt gtgttytgac tctgccaatg tgtatgccac gctgagagtg     120
   ctctccctgc cagggcaaca ccacatagag ctccaaggag accttctcca ctattcccct     180
   acggtgctgg cagtgattgg gcctgacagc accaaccgtg ctgccaccac agccgccctg     240
   ctgagccctt tcctggtgcc catgattagc tatgcggcca gcagcgagac gctcagcgtg     300
   aagcggcagt atccctcttt cctgcgcacc atccccaatg acaagtacca ggtggagacc     360
   atggtgctgc tgctgcagaa gttcgggtgg acctggatct ctctggttgg cagcagtgac     420
   gactatgggc agctaggggt gcaggcactg gagaaccagg ccactggtca ggggatctgc     480
   attgctttca aggacatcat gcccttctct gcccaggtgg gcgatgagag gatgcagtgc     540
   ctcatgcgcc acctggccca ggccggggcc accgtcgtgg ttgttttttc cagccggcag      600
   ttggccaggg tgtttttcga gtccgtggtg ctgaccaacc tgactggcaa ggtgtgggtc     660
   gcctcagaag cctgggccct ctccaggcac atcactgggg tgcccgggat ccagcgcatt     720
   gggatggtgc tgggcgtggc catccagaag agggctgtcc ctggcctgaa ggcgtttgaa     780
   gaagcctatg cccgggcaga caaggaggcc cctaggcctt gccacaaggg ctcctggtgc     840
   agcagcaatc agctctgcag agaatgccaa gctttcatgr cacacacgat gcccaagctc     900
   aaagccttct ccatgagttc tgcctacaac gcataccggg ctgtgtatgc ggtggcccat     960
   ggcctccacc agctcctggg ctgtgcctct ggagcttgtt ccaggggccg agtctacccc    1020
   tggcagcttt tggagcagat ccacaaggtg catttccttc tacacaagga cactgtggcg    1080
   tttaatgaca acagagatcc cctcagtagc tataacataa ttgcctggga ctggaatgga    1140
   cccaagtgga ccttcacggt cctcggttcc tccacatggt ctccagttca gctaaacata    1200
   aatgagacca aaatccagtg gcacggaaag gacaaccagg tgcctaagtc tgtgtgttcc    1260
   agcgactgtc ttgaagggca ccagcgagtg gttacgggtt tccatcactg ctgctttgag    1320
   tgtgtgccct gtggggctgg gaccttcctc aacaagagtg acctctacag atgccagcct    1380
   tgtgggaaag aagagtgggc acctgaggga agccagacct gcttcccgcg cactgtggtg    1440
   tttttggctt tgcgtgagca cacctcttgg gtgctgctgg cagctaacac gctgctgctg    1500
   ctgctgctgc ttgggactgc tggcctgttt gcctggcacc tagacacccc tgtggtgagg    1560
   tcagcagggg gccgcctgtg ctttcttatg ctgggctccc tggcagcagg tagtggcagc    1620
   ctctatggct tctttgggga acccacaagg cctgcgtgct tgctacgcca ggccctcttt    1680
   gcccttggtt tcaccatctt cctgtcctgc ctgacagttc gctcattcca actaatcatc    1740
   atcttcaagt tttccaccaa ggtacctaca ttctaccacg cctgggtcca aaaccacggt    1800
   gctggcctgt ttgtgatgat cagctcagcg gcccagctgc ttatctgtct aacttggctg    1860
   gtggtgtgga ccccactgcc tgctagggaa taccagcgct tcccccatct ggtgatgctt    1920
   gagtgcacag agaccaactc cctgggcttc atactggcct tcctctacaa tggcctcctc    1980
   tccatcagtg cctttgcctg cagctacctg ggtaaggact tgccagagaa ctacaacgag    2040
   gccaaatgyg tcaccttcag cctgctcttc aacttcgtgt cctggatcgc cttcttcacc    2100
   acggccagcg tctacgacgg caagtacctg cctgcggcca acatgatggc tgggctgagc    2160
   agcctgagca gcggcttcgg tgggtatttt ctgcctaagt gctacgtgat cctctgccgc    2220
   ccagacctca acagcacaga gcacttccag gcctccattc aggactacac gaggcgctgc    2280
   ggctccacct ga                                                        2292
<210> 4
   <211> 763
   <212> PRT
   <213> homo sapiens
<400> 4
   Met Arg Leu Gly Val Glu Glu Ile Asn Asn Ser Thr Ala Leu Leu Pro
    1               5                  10                  15
   Asn Ile Thr Leu Gly Tyr Gln Leu Tyr Asp Val Cys Ser Asp Ser Ala
               20                  25                  30
   Asn Val Tyr Ala Thr Leu Arg Val Leu Ser Leu Pro Gly Gln His His
           35                  40                  45
   Ile Glu Leu Gln Gly Asp Leu Leu His Tyr Ser Pro Thr Val Leu Ala
       50                  55                  60
   Val Ile Gly Pro Asp Ser Thr Asn Arg Ala Ala Thr Thr Ala Ala Leu
   65                  70                  75                  80
   Leu Ser Pro Phe Leu Val Pro Met Ile Ser Tyr Ala Ala Ser Ser Glu
                   85                  90                  95
   Thr Leu Ser Val Lys Arg Gln Tyr Pro Ser Phe Leu Arg Thr Ile Pro
               100                 105                 110
   Asn Asp Lys Tyr Gln Val Glu Thr Met Val Leu Leu Leu Gln Lys Phe
           115                 120                 125
   Gly Trp Thr Trp Ile Ser Leu Val Gly Ser Ser Asp Asp Tyr Gly Gln
       130                 135                 140
   Leu Gly Val Gln Ala Leu Glu Asn Gln Ala Thr Gly Gln Gly Ile Cys
   145                 150                 155                 160
   Ile Ala Phe Lys Asp Ile Met Pro Phe Ser Ala Gln Val Gly Asp Glu
                   165                 170                 175
   Arg Met Gln Cys Leu Met Arg His Leu Ala Gln Ala Gly Ala Thr Val
               180                 185                 190
   Val Val Val Phe Ser Ser Arg Gln Leu Ala Arg Val Phe Phe Glu Ser
           195                 200                 205
   Val Val Leu Thr Asn Leu Thr Gly Lys Val Trp Val Ala Ser Glu Ala
       210                 215                 220
   Trp Ala Leu Ser Arg His Ile Thr Gly Val Pro Gly Ile Gln Arg Ile
   225                 230                 235                 240
   Gly Met Val Leu Gly Val Ala Ile Gln Lys Arg Ala Val Pro Gly Leu
                   245                 250                 255
   Lys Ala Phe Glu Glu Ala Tyr Ala Arg Ala Asp Lys Glu Ala Pro Arg
               260                 265                 270
   Pro Cys His Lys Gly Ser Trp Cys Ser Ser Asn Gln Leu Cys Arg Glu
           275                 280                 285
   Cys Gln Ala Phe Met Ala His Thr Met Pro Lys Leu Lys Ala Phe Ser
       290                 295                 300
   Met Ser Ser Ala Tyr Asn Ala Tyr Arg Ala Val Tyr Ala Val Ala His
   305                 310                 315                 320
   Gly Leu His Gln Leu Leu Gly Cys Ala Ser Gly Ala Cys Ser Arg Gly
                   325                 330                 335
   Arg Val Tyr Pro Trp Gln Leu Leu Glu Gln Ile His Lys Val His Phe
               340                 345                 350
   Leu Leu His Lys Asp Thr Val Ala Phe Asn Asp Asn Arg Asp Pro Leu
           355                 360                 365
   Ser Ser Tyr Asn Ile Ile Ala Trp Asp Trp Asn Gly Pro Lys Trp Thr
       370                 375                 380
   Phe Thr Val Leu Gly Ser Ser Thr Trp Ser Pro Val Gln Leu Asn Ile
   385                 390                 395                 400
   Asn Glu Thr Lys Ile Gln Trp His Gly Lys Asp Asn Gln Val Pro Lys
                   405                 410                 415
   Ser Val Cys Ser Ser Asp Cys Leu Glu Gly His Gln Arg Val Val Thr
               420                 425                 430
   Gly Phe His His Cys Cys Phe Glu Cys Val Pro Cys Gly Ala Gly Thr
           435                 440                 445
   Phe Leu Asn Lys Ser Asp Leu Tyr Arg Cys Gln Pro Cys Gly Lys Glu
       450                 455                 460
   Glu Trp Ala Pro Glu Gly Ser Gln Thr Cys Phe Pro Arg Thr Val Val
   465                 470                 475                 480
   Phe Leu Ala Leu Arg Glu His Thr Ser Trp Val Leu Leu Ala Ala Asn
                   485                 490                 495
   Thr Leu Leu Leu Leu Leu Leu Leu Gly Thr Ala Gly Leu Phe Ala Trp
               500                 505                 510
   His Leu Asp Thr Pro Val Val Arg Ser Ala Gly Gly Arg Leu Cys Phe
           515                 520                 525
   Leu Met Leu Gly Ser Leu Ala Ala Gly Ser Gly Ser Leu Tyr Gly Phe
       530                 535                 540
   Phe Gly Glu Pro Thr Arg Pro Ala Cys Leu Leu Arg Gln Ala Leu Phe
   545                 550                 555                 560
   Ala Leu Gly Phe Thr Ile Phe Leu Ser Cys Leu Thr Val Arg Ser Phe
                   565                 570                 575
   Gln Leu Ile Ile Ile Phe Lys Phe Ser Thr Lys Val Pro Thr Phe Tyr
               580                 585                 590
   His Ala Trp Val Gln Asn His Gly Ala Gly Leu Phe Val Met Ile Ser
           595                 600                 605
   Ser Ala Ala Gln Leu Leu Ile Cys Leu Thr Trp Leu Val Val Trp Thr
       610                 615                 620
   Pro Leu Pro Ala Arg Glu Tyr Gln Arg Phe Pro His Leu Val Met Leu
   625                 630                 635                 640
   Glu Cys Thr Glu Thr Asn Ser Leu Gly Phe Ile Leu Ala Phe Leu Tyr
                   645                 650                 655
   Asn Gly Leu Leu Ser Ile Ser Ala Phe Ala Cys Ser Tyr Leu Gly Lys
               660                 665                 670
   Asp Leu Pro Glu Asn Tyr Asn Glu Ala Lys Cys Val Thr Phe Ser Leu
           675                 680                 685
   Leu Phe Asn Phe Val Ser Trp Ile Ala Phe Phe Thr Thr Ala Ser Val
       690                 695                 700
   Tyr Asp Gly Lys Tyr Leu Pro Ala Ala Asn Met Met Ala Gly Leu Ser
   705                 710                 715                 720
   Ser Leu Ser Ser Gly Phe Gly Gly Tyr Phe Leu Pro Lys Cys Tyr Val
                   725                 730                 735
   Ile Leu Cys Arg Pro Asp Leu Asn Ser Thr Glu His Phe Gln Ala Ser
               740                 745                 750
   Ile Gln Asp Tyr Thr Arg Arg Cys Gly Ser Thr
           755                 760
<210> 5
   <211> 1101
   <212> DNA
   <213> homo sapiens
<400> 5
   atggtgctgc tgctgcagaa gttcgggtgg acctggatct ctctggttgg cagcagtgac      60
   gactatgggc agctaggggt gcaggcactg gagaaccagg ccactggtca ggggatctgc     120
   attgctttca aggacatcat gcccttctct gcccaggtgg gcgatgagag gatgcagtgc     180
   ctcatgcgcc acctggccca ggccggggcc accgtcgtgg ttgttttttc cagccggcag      240
   ttggccaggg tgtttttcga gtccgtggtg ctgaccaacc tgactggcaa ggtgtgggtc     300
   gcctcagaag cctgggccct ctccaggcac atcactgggg tgcccgggat ccagcgcatt     360
   gggatggtgc tgggcgtggc catccagaag agggctgtcc ctggcctgaa ggcgtttgaa     420
   gaagcctatg cccgggcaga caaggaggcc cctaggcctt gccacaaggg ctcctggtgc     480
   agcagcaatc agctctgcag agaatgccaa gctttcatgr cacacacgat gcccaagctc     540
   aaagccttct ccatgagttc tgcctacaac gcataccggg ctgtgtatgc ggtggcccat     600
   ggcctccacc agctcctggg ctgtgcctct ggagcttgtt ccaggggccg agtctacccc     660
   tggcagcttt tggagcagat ccacaaggtg catttccttc tacacaagga cactgtggcg     720
   tttaatgaca acagagatcc cctcagtagc tataacataa ttgcctggga ctggaatgga     780
   cccaagtgga ccttcacggt cctcggttcc tccacatggt ctccagttca gctaaacata     840
   aatgagacca aaatccagtg gcacggaaag gacaaccagg tgcctaagtc tgtgtgttcc     900
   agcgactgtc ttgaagggca ccagcgagtg gttacgggtt tccatcactg ctgctttgag     960
   tgtgtgccct gtggggctgg gaccttcctc aacaagagtt attcctactc tgctcatctg    1020
   gctctcagga accttcttgg ctcttcctct ttcagacctc tacagatgcc agccttgtgg    1080
   gaaagaagag tgggcacctg a                                              1101
<210> 6
   <211> 366
   <212> PRT
   <213> homo sapiens
<400> 6
   Met Val Leu Leu Leu Gln Lys Phe Gly Trp Thr Trp Ile Ser Leu Val
    1               5                  10                  15
   Gly Ser Ser Asp Asp Tyr Gly Gln Leu Gly Val Gln Ala Leu Glu Asn
               20                  25                  30
   Gln Ala Thr Gly Gln Gly Ile Cys Ile Ala Phe Lys Asp Ile Met Pro
           35                  40                  45
   Phe Ser Ala Gln Val Gly Asp Glu Arg Met Gln Cys Leu Met Arg His
       50                  55                  60
   Leu Ala Gln Ala Gly Ala Thr Val Val Val Val Phe Ser Ser Arg Gln
   65                  70                  75                  80
   Leu Ala Arg Val Phe Phe Glu Ser Val Val Leu Thr Asn Leu Thr Gly
                   85                  90                  95
   Lys Val Trp Val Ala Ser Glu Ala Trp Ala Leu Ser Arg His Ile Thr
               100                 105                 110
   Gly Val Pro Gly Ile Gln Arg Ile Gly Met Val Leu Gly Val Ala Ile
           115                 120                 125
   Gln Lys Arg Ala Val Pro Gly Leu Lys Ala Phe Glu Glu Ala Tyr Ala
       130                 135                 140
   Arg Ala Asp Lys Glu Ala Pro Arg Pro Cys His Lys Gly Ser Trp Cys
   145                 150                 155                 160
   Ser Ser Asn Gln Leu Cys Arg Glu Cys Gln Ala Phe Met Ala His Thr
                   165                 170                 175
   Met Pro Lys Leu Lys Ala Phe Ser Met Ser Ser Ala Tyr Asn Ala Tyr
               180                 185                 190
   Arg Ala Val Tyr Ala Val Ala His Gly Leu His Gln Leu Leu Gly Cys
           195                 200                 205
   Ala Ser Gly Ala Cys Ser Arg Gly Arg Val Tyr Pro Trp Gln Leu Leu
       210                 215                 220
   Glu Gln Ile His Lys Val His Phe Leu Leu His Lys Asp Thr Val Ala
   225                 230                 235                 240
   Phe Asn Asp Asn Arg Asp Pro Leu Ser Ser Tyr Asn Ile Ile Ala Trp
                   245                 250                 255
   Asp Trp Asn Gly Pro Lys Trp Thr Phe Thr Val Leu Gly Ser Ser Thr
               260                 265                 270
   Trp Ser Pro Val Gln Leu Asn Ile Asn Glu Thr Lys Ile Gln Trp His
           275                 280                 285
   Gly Lys Asp Asn Gln Val Pro Lys Ser Val Cys Ser Ser Asp Cys Leu
       290                 295                 300
   Glu Gly His Gln Arg Val Val Thr Gly Phe His His Cys Cys Phe Glu
   305                 310                 315                 320
   Cys Val Pro Cys Gly Ala Gly Thr Phe Leu Asn Lys Ser Tyr Ser Tyr
                   325                 330                 335
   Ser Ala His Leu Ala Leu Arg Asn Leu Leu Gly Ser Ser Ser Phe Arg
               340                 345                 350
   Pro Leu Gln Met Pro Ala Leu Trp Glu Arg Arg Val Gly Thr
           355                 360                 365
<210> 7
   <211> 705
   <212> DNA
   <213> homo sapiens
<400> 7
   atgctgggct ccctggcagc aggtagtggc agcctctatg gcttctttgg ggaacccaca     60
   aggcctgcgt gcttgctacg ccaggccctc tttgcccttg gtttcaccat cttcctgtcc    120
   tgcctgacag ttcgctcatt ccaactaatc atcatcttca agttttccac caaggtacct    180
   acattctacc acgcctgggt ccaaaaccac ggtgctggcc tgtttgtgat gatcagctca    240
   gcggcccagc tgcttatctg tctaacttgg ctggtggtgt ggaccccact gcctgctagg    300
   gaataccagc gcttccccca tctggtgatg cttgagtgca cagagaccaa ctccctgggc    360
   ttcatactgg ccttcctcta caatggcctc ctctccatca gtgcctttgc ctgcagctac    420
   ctgggtaagg acttgccaga gaactacaac gaggccaaat gygtcacctt cagcctgctc    480
   ttcaacttcg tgtcctggat cgccttcttc accacggcca gcgtctacga cggcaagtac    540
   ctgcctgcgg ccaacatgat ggctgggctg agcagcctga gcagcggctt cggtgggtat    600
   tttctgccta agtgctacgt gatcctctgc cgcccagacc tcaacagcac agagcacttc    660
   caggcctcca ttcaggacta cacgaggcgc tgcggctcca cctga                    705
<210> 8
   <211> 234
   <212> PRT
   <213> homo sapiens
<400> 8
   Met Leu Gly Ser Leu Ala Ala Gly Ser Gly Ser Leu Tyr Gly Phe Phe
   1               5                   10                  15
   Gly Glu Pro Thr Arg Pro Ala Cys Leu Leu Arg Gln Ala Leu Phe Ala
               20                  25                  30
   Leu Gly Phe Thr Ile Phe Leu Ser Cys Leu Thr Val Arg Ser Phe Gln
           35                  40                  45
   Leu Ile Ile Ile Phe Lys Phe Ser Thr Lys Val Pro Thr Phe Tyr His
       50                  55                  60
   Ala Trp Val Gln Asn His Gly Ala Gly Leu Phe Val Met Ile Ser Ser
   65                  70                  75                  80
   Ala Ala Gln Leu Leu Ile Cys Leu Thr Trp Leu Val Val Trp Thr Pro
                   85                  90                  95
   Leu Pro Ala Arg Glu Tyr Gln Arg Phe Pro His Leu Val Met Leu Glu
               100                 105                 110
   Cys Thr Glu Thr Asn Ser Leu Gly Phe Ile Leu Ala Phe Leu Tyr Asn
           115                 120                 125
   Gly Leu Leu Ser Ile Ser Ala Phe Ala Cys Ser Tyr Leu Gly Lys Asp
       130                 135                 140
   Leu Pro Glu Asn Tyr Asn Glu Ala Lys Cys Val Thr Phe Ser Leu Leu
   145                 150                 155                 160
   Phe Asn Phe Val Ser Trp Ile Ala Phe Phe Thr Thr Ala Ser Val Tyr
                   165                 170                 175
   Asp Gly Lys Tyr Leu Pro Ala Ala Asn Met Met Ala Gly Leu Ser Ser
               180                 185                 190
   Leu Ser Ser Gly Phe Gly Gly Tyr Phe Leu Pro Lys Cys Tyr Val Ile
           195                 200                 205
   Leu Cys Arg Pro Asp Leu Asn Ser Thr Glu His Phe Gln Ala Ser Ile
       210                 215                 220
   Gln Asp Tyr Thr Arg Arg Cys Gly Ser Thr
   225                 230
<210> 9
   <211> 2951
   <212> DNA
   <213> homo sapiens
<400> 9
   gtcactgggt gccacctggt ttgcatctgt gccttcgtcc tgcccagttc ctgagtggga    60
   ccgcaggccc ggaatgtcaa ggcaaacagt cctgcttcag ccactgggct ccagtcccac   120
   ccctttgggg ggcctgaagt taggaagcat ccggcagctg ccttctattt aagcaactgg   180
   cctccttaga ggccactcct tggccatgcc aggcgcgggc atctggccag catgctgctc   240
   tgcacggctc gcctggtcgg cctgcagctt ctcatttcct gctgctgggc ctttgcctgc   300
   catagcacgg agtcttctcc tgacttcacc ctccccggag attacctcct ggcaggcctg   360
   ttccctctcc attctggctg tctgcaggtg aggcacagac ccgaggtgac cctgtgtgac   420
   aggtcttgta gcttcaatga gcatggctac cacctcttcc aggctatgcg gcttggggtt   480
   gaggagataa acaactccac ggccctgctg cccaacatca ccctggggta ccagctgtat   540
   gatgtgtgtt ytgactctgc caatgtgtat gccacgctga gagtgctctc cctgccaggg   600
   caacaccaca tagagctcca aggagacctt ctccactatt cccctacggt gctggcagtg   660
   attgggcctg acagcaccaa ccgtgctgcc accacagccg ccctgctgag ccctttcctg   720
   gtgcccatga ttagctatgc ggccagcagc gagacgctca gcgtgaagcg gcagtatccc   780
   tctttcctgc gcaccatccc caatgacaag taccaggtgg agaccatggt gctgctgctg   840
   cagaagttcg ggtggacctg gatctctctg gttggcagca gtgacgacta tgggcagcta   900
   ggggtgcagg cactggagaa ccaggccact ggtcagggga tctgcattgc tttcaaggac   960
   atcatgccct tctctgccca ggtgggcgat gagaggatgc agtgcctcat gcgccacctg  1020
   gcccaggccg gggccaccgt cgtggttgtt ttttccagcc ggcagttggc cagggtgttt  1080
   ttcgagtccg tggtgctgac caacctgact ggcaaggtgt gggtcgcctc agaagcctgg  1140
   gccctctcca ggcacatcac tggggtgccc gggatccagc gcattgggat ggtgctgggc  1200
   gtggccatcc agaagagggc tgtccctggc ctgaaggcgt ttgaagaagc ctatgcccgg  1260
   gcagacaagg aggcccctag gccttgccac aagggctcct ggtgcagcag caatcagctc  1320
   tgcagagaat gccaagcttt catgrcacac acgatgccca agctcaaagc cttctccatg  1380
   agttctgcct acaacgcata ccgggctgtg tatgcggtgg cccatggcct ccaccagctc  1440
   ctgggctgtg cctctggagc ttgttccagg ggccgagtct acccctggca gcttttggag  1500
   cagatccaca aggtgcattt ccttctacac aaggacactg tggcgtttaa tgacaacaga  1560
   gatcccctca gtagctataa cataattgcc tgggactgga atggacccaa gtggaccttc  1620
   acggtcctcg gttcctccac atggtctcca gttcagctaa acataaatga gaccaaaatc  1680
   cagtggcacg gaaaggacaa ccaggtgcct aagtctgtgt gttccagcga ctgtcttgaa  1740
   gggcaccagc gagtggttac gggtttccat cactgctgct ttgagtgtgt gccctgtggg  1800
   gctgggacct tcctcaacaa gagtgacctc tacagatgcc agccttgtgg gaaagaagag  1860
   tgggcacctg agggaagcca gacctgcttc ccgcgcactg tggtgttttt ggctttgcgt  1920
   gagcacacct cttgggtgct gctggcagct aacacgctgc tgctgctgct gctgcttggg  1980
   actgctggcc tgtttgcctg gcacctagac acccctgtgg tgaggtcagc agggggccgc  2040
   ctgtgctttc ttatgctggg ctccctggca gcaggtagtg gcagcctcta tggcttcttt    2100
   ggggaaccca caaggcctgc gtgcttgcta cgccaggccc tctttgccct tggtttcacc    2160
   atcttcctgt cctgcctgac agttcgctca ttccaactaa tcatcatctt caagttttcc    2220
   accaaggtac ctacattcta ccacgcctgg gtccaaaacc acggtgctgg cctgtttgtg    2280
   atgatcagct cagcggccca gctgcttatc tgtctaactt ggctggtggt gtggacccca    2340
   ctgcctgcta gggaatacca gcgcttcccc catctggtga tgcttgagtg cacagagacc    2400
   aactccctgg gcttcatact ggccttcctc tacaatggcc tcctctccat cagtgccttt    2460
   gcctgcagct acctgggtaa ggacttgcca gagaactaca acgaggccaa atgygtcacc    2520
   ttcagcctgc tcttcaactt cgtgtcctgg atcgccttct tcaccacggc cagcgtctac    2580
   gacggcaagt acctgcctgc ggccaacatg atggctgggc tgagcagcct gagcagcggc    2640
   ttcggtgggt attttctgcc taagtgctac gtgatcctct gccgcccaga cctcaacagc    2700
   acagagcact tccaggcctc cattcaggac tacacgaggc gctgcggctc cacctgacca    2760
   gtgggtcagc aggcacggct ggcagccttc tctgccctga gggtcgaagg tcgagcaggc    2820
   cgggggtgtc cgggaggtct ttgggcatcg cggtctgggg ttgggacgtg taagcgcctg    2880
   ggagagccta gaccaggctc cgggctgcca ataaagaagt gaaatgcgwa aaaaaaaaaa    2940
   aaaaaaaaaa a                                                         2951